# EUROPEAN PATENT APPLICATION

(11) **EP 1 457 500 A1**
(43) Date of publication of application: **15.09.2004**
(21) Application number: 03005829.1
(22) Date of filing: 14.03.2003
(51) Int. Cl.: C07K 19/00, C07K 14/47, G01N 33/68, C12N 5/10, C12N 15/62, C12N 15/63, G01N 33/50, A61K 35/28, A01K 67/027, A61K 38/17

(54) **Soluble hybrid prion proteins and their use in the diagnosis, prevention and treatment of transmissible spongiform encephalophathies**

(71) Applicant: University of Zurich, 8006 Zürich (CH)
(72) Inventor: Aguzzi, Adriano, 8001 Zürich (CH); Genoud, Nicolas, 1618 Châtel-St-Denis (CH); Raeber, Alex, 8046 Zürich (CH)
(74) Representative: Grünecker, Kinkeldey, Stockmair & Schwanhäusser Anwaltssozietät

(57) **Abstract**

The present invention relates to a soluble hybrid protein, comprising at least a first polypeptide sequence derived from a prion protein PrP^{c} that is capable of binding a protein responsible for transmissible spongiform encephalitis (PrP^{sc}), and a second polypeptide sequence (tag), wherein said hybrid protein does not comprise a functional membrane anchor moiety. Also, the present invention is directed to the use of said hybrid proteins for the diagnosis of transmissible spongiform encephalopathies. In addition, the present invention relates to the use of said hybrid protein, a nucleic acid encoding said hybrid protein, a vector, and/or a host cell comprising a nucleic acid encoding said hybrid protein for the preparation of a medicament for the prevention or treatment of transmissible spongiform encephalopathies (TSEs). Furthermore, the present invention is directed to a process for producing said hybrid proteins and it also relates to transgenic animals stably expressing said hybrid protein, the bone marrow of said transgenic animals and the use of said bone marrow for the treatment of transmissible spongiform encephalopathies (TSEs).

## Description

### FIELD OF THE INVENTION

The present invention relates to a soluble hybrid protein, comprising at least a first polypeptide sequence derived from a prion protein PrP^{c} that is capable of binding a protein responsible for transmissible spongiform encephalitis (PrP^{sc}), and a second polypeptide sequence (tag), wherein said hybrid protein does not comprise a functional membrane anchor moiety. Also, the present invention is directed to the use of said hybrid proteins for the diagnosis of transmissible spongiform encephalopathies. In addition, the present invention relates to the use of said hybrid protein, a nucleic acid encoding said hybrid protein, a vector, and/or a host cell comprising a nucleic acid encoding said hybrid protein for the preparation of a medicament for the prevention and/or treatment of transmissible spongiform encephalopathies (TSEs). Furthermore, the present invention is directed to a process for producing said hybrid proteins and it also relates to transgenic animals stably expressing said hybrid protein, the bone marrow of said transgenic animals and the use of said bone marrow for the treatment of transmissible spongiform encephalopathies (TSEs).

### BACKGROUND OF THE INVENTION

Transmissible spongiform encephalopathies (TSEs) are fatal neurodegenerative diseases that affect mammals including cattle (BSE), human (Creutzfeldt-Jakob-Disease) and sheep (scrapie). The disease is characterized by the accumulation of an abnormal partially protease-resistant form (PrP-res or PrP^{sc}) of the host-encoded cellular prion protein (PrP^{c}). It has been postulated that the infectious agent or prion is partly or entirely composed of PrP^{sc} and that it propagates itself by inducing conversion of PrP^{c} to PrP^{sc} (Prusiner, 1982). The three-dimensional structure in solution of the normal, protease sensitive form of PrP (PrP^{c}) from various species has been elucidated and it exists considerable evidence that PrP^{sc} represents an abnormal conformational form with significantly increased ß-sheet content. The ß-sheet rich conformation is prone to aggregation and forms amyloid fibrils accumulating in distinct amyloid plaques in brain tissue (for review see Aguzzi et al. 2001 b).

A wealth of evidence indicates that PrP^{c} is essential for the development of prion disease: ablation of the *Prnp* gene which encodes PrP^{c} (Büeler et al., 1992) confers resistance to experimental scrapie inoculation (Büeler et al., 1993), and all familial cases of human transmissible spongiform encephalopathies (TSEs) are characterized by mutations of *Prnp.*

The open reading frame encoding PrP^{c} is contained within a single exon of the *Prnp* gene. PrP^{c} is processed into a mature form by removal of its amino terminal secretory signal sequence, glycosylation, and replacement of its carboxy-terminal signal sequence with a GPI residue which anchors it to the outer cell surface. The physiological function of PrP^{c} is still poorly understood. Whilst the majority of PrP^{c} is membrane bound, a soluble form of PrP^{c} lacking the glycolipid moiety is present in serum (Perini et al., 1996) and epididymal fluid (Gatti et al., 2002). In primary cultures of splenocytes and cerebellar granule cells, a substantial amount of PrP^{c} is shed after loss of the GPI anchor. Shedding is increased in the presence of serum, suggesting that serum phospholipases participate in'this process (Parizek et al., 2001).

Most theoretical models of prion amplification predict that PrP^{sc} and PrP^{c} interact directly in the course of TSE pathogenesis. In cell-free conversion systems, PrP^{sc} drives conversion of PrP^{c} into a moiety that shares many physico-chemical properties with PrP^{sc} (Kocisko et al., 1994). These include aggregation, formation of fibrils and partial resistance to digestion with proteinase K (PK). Specific binding of protease-resistant PrP^{sc} to protease-sensitive PrP^{c} has been observed in radiolabelled lysates of cells expressing soluble PrP^{c} that lack the GPI anchor (Horiuchi and Caughey, 1999). Animal studies (Scott et al., 1989) and *in vitro* conversion assays (Bessen et al., 1995; Kocisko et al., 1995) indicate that sequence compatibility between PrP^{c} and PrP^{sc} is necessary for efficient PrP^{sc} formation.

Cell-free systems indicate that binding of PrP^{c} to PrP^{sc} and conversion to the protease-, resistant state are kinetically separable (DebBurman et al., 1997). The initial binding between the two partners is a critical aspect of the overall conversion mechanism. The conversion event has been proposed to occur either on the cell surface or during the endocytic trafficking of PrP^{sc}. PrP^{sc} then appears to accumulate in lysosomes. However, others find that disease-associated PrP accumulates mainly extracellularly or on neu-, ronal surfaces in infected animals (Jeffrey et al., 1992) and cultured cells (Vey et al., 1996), suggesting that conversion may be primarily extracellular.

Technical obstacles have prevented the demonstration of physical interaction between PrP^{c} and PrP^{sc} *in vivo -* a central prediction of the protein-only hypothesis. When introduced from the outside, PrP^{sc} is rapidly degraded (Sailer et al., 1994). Hence, it is not possible to determine its biochemical fate directly.

It is one objective of the present invention to provide new diagnostic agents suitable for the detailed quantitative and qualitative analysis of PrP^{sc} and PrP^{c}-PrP^{sc} complexes in *vitro* and *in vivo.*

Furthermore, there is an urgent need for efficiently and safely screening TSEs in the preclinical state by detecting low concentrations of PrP^{sc} in peripheral and accessible tissues (e.g. tonsils) or body fluids (e.g. blood, urine, cerebrospinal fluid).

In particular there is a need for compounds for the prevention and treatment of TSE's that inhibit the formation of PrP^{sc} and/or sequester PrP^{sc}.

### Disclosure of the invention

One aspect of the present invention provides a soluble hybrid protein, comprising at least
a) a first polypeptide sequence derived from a prion protein PrP^{c} that is capable of binding a protein responsible for transmissible spongiform encephalitis (PrP^{sc}),
b) and a second polypeptide sequence (tag),
wherein said hybrid protein does not comprise a functional membrane anchor moiety.

The first polypeptide sequence according to the invention is defined herein as any prion protein PrP^{c} or a functional derivative thereof that is capable of binding a protein responsible for transmissible spongiform encephalitis (PrP^{sc}).

The term "functional derivative" of a prion protein PrP^{c} encompasses polypeptides that are preferably at least 50%, more preferably at least 80%, and most preferably at least 90% identical to the amino acid sequence of the human biologically active and naturally occurring, fully functional prion protein PrP^{c}, i.e. mature (posttranslationally processed, without the amino terminal secretory sequence) wild-type human prion protein PrP^{c} (Schätzl et al., J. Mol. Biol. 245, 362-374 (1995)), or a functional fragment (partial prion protein PrP^{c} amino acid sequence with prion protein PrP^{sc} binding activity) thereof, and which retain at least some, preferably at least 20%, more preferably at least 50%, and most preferably at least 90% of the PrP^{sc} binding activity of the of the wild-type prion protein PrP^{c}.

% Identity of mutually related polypeptides can be determined by means of known procedures. As a rule, special computer programs with algorithms taking account of the special requirements are used. Preferred procedures for the determination of % identity firstly generate the greatest agreement between the sequences studied. Computer programs for the determination of the identity between two sequences include, but are not limited to, the GCG program package, including GAP (Devereux J et al., Nucleic Acids Research 12 (12): 387 (1984); Genetics Computer Group University of Wisconsin, Madison (WI); BLASTP, BLASTN and FASTA (Altschul S et al., J. Molec. Biol. 215: 403-410 (1990)). The BLAST X program can be obtained from the National Centre for Biotechnology Information (NCBI) and from other sources (BLAST Handbook, Altschul S et al., NCB NLM NIH Bethesda MD 20894; Altschul S et al., J. Mol. 215: 403-410 (1990)). The well-known Smith Waterman algorithm can also be used for the determination of % identity.

Preferred parameters for the sequence comparison include the following:

| | |
|---|---|
| Algorithm | Needleman and Wunsch, J. Mol. Biol. 48: 443-453 (1970) |
| Comparison matrix | BLOSUM62 from Henikoff & Henikoff, PNAS USA 89 (1992), 10915-10919 |
| Gap penalty | 12 |
| Gap-length penalty | 2 |

The GAP program is also suitable for use with the above parameters. The above parameters are the standard parameters (default parameters) for amino acid sequence comparisons, in which gaps at the ends do not decrease the % identity value. With very small sequences compared to the reference sequence, it can further be necessary to increase the expectancy value to up to 100,000 and in some cases to reduce the word length (word size) to down to 2.

Further model algorithms, gap opening penalties, gap extension penalties and comparison matrices including those named in the Program Handbook, Wisconsin Package, Version 9, September 1997, can be used. The choice will depend on the comparison to be performed and further on whether the comparison is performed between sequence pairs; where GAP or Best Fit are preferred, or between one sequence and a large sequence database, where FASTA or BLAST are preferred.

The above polypeptide based prion protein PrP^{c} derivatives include functional fragments (a partial prion protein PrP^{c} amino acid sequence with prion protein PrP^{sc} binding activity) and variants (e.g., structurally and biologically similar to the wild-type protein and having PrP^{sc} binding activity). Furthermore, the term "prion protein PrP^{c} derivative" also includes prion protein PrP^{c} analogs (e.g., a protein or fragment thereof that is substantially similar in PrP^{sc} binding to the wild-type protein or a fragment thereof), chemical derivatives of prion protein PrP^{c} (e.g., contains additional chemical moieties, such as polyethyleneglycol and derivatives thereof), and peptidomimetics of prion protein PrP^{c} (e.g., a low molecular weight compound that mimics a peptide in structure and/or function (see, e.g., Abell, *Advances in Amino Acid Mimetics and Peptidomimetics,* London: JAI Press (1997); Gante, *Peptidmimetica* - *massgeschneiderte Enzyminhibitoren Angew. Chem.* 106: 1780-1802 (1994); and Olson et al., *J. Med. Chem.* 36: 3039-3049 (1993)), all of which retain at least some, preferably at least 20%, more preferably at least 50%, and most preferably at least 90% of the PrP^{sc} binding function of the human mature wild-type prion protein PrP^{c}, which binding function can be tested in any established binding assay in the prior art, such as a luminescence immunoassay (Biffiger et al., *J Virol Methods* 101: 79-84 (2002)). Preferred functional derivatives contain a substitution, deletion or addition of at least one amino acid.

In a preferred embodiment, the hybrid protein according to the invention is one, wherein the first polypeptide sequence is a functional derivative of the human prion protein (PrP^{c}) or a functional fragment thereof, comprising the PrP^{sc} binding domain of human PrP^{c} that is preferably at least 50%, more preferably at least 80%, and most preferably at least 90% identical to the PrP^{sc} binding domain of the human naturally occurring, fully functional, wild-type prion protein PrP^{c}, and retains at least some, preferably at least 20%, more preferably at least 50%, and most preferably at least 90% of the specific binding capability to a PrP^{sc}-protein of the PrP^{sc} binding domain of the wild-type prion protein.

More preferably, the hybrid protein is one, wherein the functional derivative of the wild-type prion protein is a functional fragment, variant, analog, chemical derivative, or peptidomimetic of the wild-type prion protein or a functional fragment thereof.

PrP genes encoding PrP prion proteins have been elucidated in many mammalian species. For example, some commonly known PrP sequences are described in Schätzl et al., J. Mol. Biol. 245, 362-374 (1995); Wopfner et al., J. Mol .Biol. 289, 1163-1178 (1999), and the corresponding sequences are deposited at Genbank accessible via the Public Medline database. The protein expressed by such a gene can assume either a PrP^{c} (non-disease) or PrP^{sc} (disease) form.

The terms "prion protein PrP^{c}" or "PrP^{c}" in short designate the naturally occurring form of the mature Pmp gene product. PrP^{c} is processed into a mature form by removal of its amino terminal secretory signal sequence, glycosylation, and replacement of its carboxy-terminal signal sequence with a GPI residue. Its presence in a given cell type is necessary, but not sufficient for prion replication.

The term PrP^{sc} designates an "abnormal" form of the mature Pmp gene product found in tissues of TSE sufferers. This form is defined as being partially resistant to protease digestion under standardized conditions (see Schaller et al., *Acta Neuropathol* (Berl) 98: 437-443 (1999)). PrP^{sc} is believed to differ from PrP^{c} only (or mainly) conformationally.

The hybrid proteins according to the present invention are useful for *in vitro* and *in vivo* applications such as diagnosis, prophylaxis, and therapy.

The hybrid proteins are capable of binding stably to the PrP^{sc} form of a prion protein. The hybrid bound PrP^{sc} form of prion protein is thus labeled for further diagnostic measures or for being detected by a mammals immune system.

For therapeutic and prophylactic *in vivo* applications, it is preferred that the hybrid protein of the invention is one, which is resistant to conversion to the PrP^{sc} form when binding to PrP^{sc}.

In a preferred embodiment the hybrid proteins according to the invention form multimeric hybrid proteins, preferably dimers or pentamers, more preferably dimers. Multimeric hybrid proteins are more stable in structure and/or binding than monomeric hybrid proteins.

Preferably, it is the second polypeptide sequence that is capable of forming a covalent linkage (such as disulfide bridge) or altemative binding (e.g. chemical cross linking) to at least one further second polypeptide sequence of at least one further hybrid protein to form multimeric hybrid proteins.

For applications in membrane containing environments it is important that the hybrid protein does not comprise a functional membrane anchor moiety so as to avoid immobilization by binding to membranes before reaching the desired binding partner.

As referred herein, the term "functional membrane anchor moiety" refers to an amino acid sequence that attaches a given polypeptide containing the same to lipid membranes *in vitro* and/or *in vivo,* preferably to cell membranes *in vitro* and/or *in vivo.*

More preferably, the hybrid protein comprises a first polypeptide sequence that does not comprise the GPI-(Glycosyl-phosphatidyl-Inositol) residue and/or the carboxy-terminal signal sequence of the wild type PrP^{c} prion protein.

In a preferred embodiment the present invention is directed to a multimeric hybrid protein, comprising dimers or oligomers of any one of the hybrid proteins of the invention, preferably dimers or pentamers.

In the multimeric hybrid protein the first and the second polypeptide sequence of a hybrid protein can be derived from the same or different species. For example, the first polypeptide sequence can be derived from one mammal and the second polypeptide sequence can be derived from another. More preferably, both polypeptide sequences are derived from the same mammals; most preferably, both sequences are of human origin.

Also, in the multimeric hybrid proteins the monomeric units can be derived from the same (homo multimer) or different (hetero multimer) species. For example, one hybrid monomer can be of bovine (or of bovine and a further origin, see section above) origin while another hybrid monomer is of human (or of human and a further origin, see section above) origin. Preferably, the monomeric units of the multimeric hybrid proteins are derived from the same species.

In a more preferred embodiment of the present invention the hybrid protein of the invention is one, wherein the first polypeptide sequence is a full length prion protein (PrP^{c}), more preferably a full-length prion protein (PrP^{c}) that does not comprise the GPI-(Glycosyl-phosphatidyl-Inositol) residue and/or the carboxy-terminal signal sequence of the wild type PrP^{c} prion protein.

The term "full length prion protein (PrP^{c})" refers to the amino acid sequence encoded by a naturally occurring PrnP gene after removal of the amino terminal secretory signal sequence with or without further glycosylation.

The second polypeptide sequence of the hybrid proteins is preferably linked by a peptidic bond to the first polypeptide sequence but other linkers can also be employed for this purpose (e.g. formaldehyde, glutaraldehyde). Non peptidic polypeptide linkers are commonly known among those skilled in the art, e.g. the streptavidin-biotin linkage system.

It has been found that the second polypeptide sequence stabilizes the structural conformation of the first sequence. Furthermore, the second polypeptide sequence may also function to tag the protein so that specific diagnostic reagents can interact with said tag to detect and/or separate the hybrid protein and also to detect and/or separate its binding partner PrP^{sc}. For example, the second sequence polypeptide can comprise a reactive moiety, such as, e.g. a biotin, that can be captured by a reactive counterpart, such as, e.g. a streptavidin containing diagnostic reagent, to detect and/or bind said hybrid protein alone or together with its binding partner PrP^{sc}. Hybrid proteins with or without PrP^{sc} binding partners can be differentiated by standard protein techniques, e.g. by the difference in size in a PAGE gel system in the presence of known molecular weight markers. Moreover, in *in vivo* systems, i.e. in a mammal, the tag can elicit an immunological response to the hybrid protein so that is sequestered together with any bound PrP^{sc} by the immune system and thus assists the destruction of dangerous PrP^{sc} prion proteins.

In a particularly preferred embodiment, the second polypeptide sequence according to the invention is derived from an immunoglobulin. Preferably, said derived immunoglobulin is a functional derivative of an immunoglobulin.

The function of said derivative may be one that is typical for immunoglobulines such as multimer formation, epitope binding and/or complement activation. Preferably, the derivative should not induce an immune response.

The term "functional derivative" of an immunoglobulin encompasses polypeptides that are preferably at least 50%, more preferably at least 80%, and most preferably at least 90% identical to the amino acid sequence of a biologically active and naturally occurring, fully functional immunoglobulin, i.e. a wild-type mammalian immunoglobulin or a functional fragment (a partial immunoglobulin amino acid sequence with immunoglobulin binding and/or complement related activity) thereof, preferably the human immunoglobulin gamma or mµ heavy chain (Canfield et al., *J Exp Med* 173: 1483-1491 (1991), Lund et al., *J Immunol 147:* 2657-2662 (1991) and Tao et al., *J Exp Med* 178: 661-667 (1993)), and which retain at least some, preferably at least 20%, more preferably at least 50%, and most preferably at least 90% of the immunoglobulin binding activity (i.e. binding activity towards other immunoglobulins or fragments thereof, and/or complement factors, preferably binding towards other immunoglobulins for multimer formation) and/or complement related activity of the wild-type immunoglobulin.

The above polypeptide based immunoglobulin derivatives include functional fragments (partial immunoglobulin amino acid sequence with immunoglobulin binding and/or complement related activity) and variants (e.g., structurally and biologically similar to the wild-type immunoglobulin and having at least one biologically equivalent domain of an immunoglobulin). Furthermore, the term "immunoglobulin derivative" also includes immunoglobulin analogs (e.g., a protein or fragment thereof substantially similar in biological function to the wild-type protein or fragment thereof), chemical derivatives of an immunoglobulin (e.g., contains additional chemical moieties, such as polyethyleneglycol and derivatives thereof), and peptidomimetics of an immunoglobulin (e.g., a low molecular weight compound that mimics a peptide in structure and/or function (see, e.g., Abell, *Advances in Amino Acid Mimetics and Peptidomimetics,* London: JAI Press (1997);Gante, *Peptidmimetica - massgeschneiderte Enzyminhibitoren Angew. Chem.* 106: 1780-1802 (1994); and Olson et al., *J. Med. Chem.* 36: 3039-3049 (1993)), all of which retain at least some, preferably at least 20%, more preferably at least 50%, and most preferably at least 90% of the immunoglobulin binding and/or complement related function of the wild-type immunoglobulin.

More preferably, in the second polypeptide sequence the binding sites for Fc_{γ} receptors and for complement are inactivated.

Even more preferably, the hybrid protein according to the invention is one, wherein said second polypeptide sequence is derived from an immunoglobulin heavy chain constant region, most preferably is derived from a mammalian immunoglobulin gamma heavy chain, preferably from the human immunoglobulin gamma heavy chain.

Also more preferred is a hybrid protein, wherein said second polypeptide sequence is derived from a mammalian immunoglobulin mµ heavy chain, preferably from the human immunoglobulin mµ heavy chain.

Further, the second polypeptide sequence may contain a FK506-binding protein (FKBP)-domain promoting the binding to at least one further second polypeptide sequence of at least one further hybrid protein to form dimeric/multimeric hybrid proteins (Briesewitz et al., 1999).

Preferably, the hybrid protein is one, wherein said second polypeptide sequence is fused directly to the carboxy-terminal side of the first polypeptide sequence.

In a preferred embodiment, the hybrid protein is one that is bound to a solid support. Thus the hybrid protein and optionally its binding partner PrP^{sc} may easily be isolated from other compounds and analyzed. Suitable solid support materials for proteins are well known among those skilled in the art.

Because the hybrid proteins bind stably to PrP^{sc} they are particularly useful for diagnosis.

Therefore, in a further aspect the present invention relates to the use of a hybrid protein according to the invention for the diagnosis of transmissible spongiform encephalopathies (TSEs).

The present invention further provides diagnostic kits for the use in the diagnosis of transmissible spongiform encephalopathies (TSEs).

In another aspect the present invention relates to a process for the detection of transmissible spongiform encephalopathies (TSEs). Preferably, said process comprises the steps of
(a) contacting a sample suspected of comprising a PrP^{sc} protein with a hybrid protein according to the invention under conditions that allow for the binding of the PrP^{sc} protein to the hybrid protein, and
(b) detecting the PrP^{sc} protein-hybrid protein complex.

The present invention also relates to a process for identifying and/or isolating molecules capable of directly or indirectly binding to PrP^{sc} and/or PrP^{c}. Preferably, said process compnses the following steps
(a) contacting a sample suspected of comprising a PrP^{sc} and/or PrP^{c} binding molecule with a hybrid protein according to any one of claims 1 to 18 under conditions that allow for the binding of the molecule to the hybrid protein either directly or indirectly, and
(b) detecting whether the hybrid protein has bound a molecule, and optionally
(c) isolating the molecule directly or indirectly bound to the hybrid protein.

The hybrid protein according to the invention can further be used for the identification of molecules interacting with PrP^{sc} and/or PrP^{c}.

In a further preferred embodiment of the invention soluble hybrid pnon protein, preferably produced in cultured cells and preferably affinity-immobilized onto beads, efficiently captures PrP^{sc} from tissue homogenates of TSE-infected mammals, preferably humans.

It is an important advantage of the diagnostic TSE assay of the present invention that no protease treatment is necessary to distinguish the disease related conformation of the prion protein PrP^{sc} from its normal homologue PrP^{c}.

Preferably, a tissue sample is derived from brain or other tissues known to express the prion protein, like lymphoid tissues, e.g tonsils. Also preferred are samples from body fluids like blood, saliva, urine or cerebrospinal fluid.

The parameters for the diagnostic application of the hybrid proteins such as the auxiliary reagents (e.g. solid phase, tag recognition reagent, etc.), buffer solutions, pH, temperature, incubation time, method of detection, etc. will vary with each hybrid system, the specific tag, and the assayed tissue or fluid. The skilled person is capable of determining and optimizing these parameters through routine experimentation in view of the abundant prior art on polypeptide diagnostic methods. Additionally, the appended examples assist the skilled person to determine and optimize these parameters.

For detection purposes, the hybrid protein according to the invention may contain any label, such as radioisotopes, fluorescent dyes, and luminescent dyes including electrochemoluminescent dyes.

A further aspect of the present invention relates to the nucleic acid molecules encoding a hybrid protein according to the invention. The nucleic acid sequence of the hybrid proteins may be directly deduced from the amino acid sequence, or more conveniently, be already available because the hybrid proteins are produced recombinantly. For recombinant production the DNA encoding the first and second polypeptide sequence can be obtained and modified as desired by chemical DNA, synthesis or DNA amplification or molecular cloning directly from a tissue, cell culture, or cloned DNA using standard molecular biology techniques (Sambrook et al. 1969, Molecular Cloning - A Laboratory Manual, 2^{nd} Ed., Cold Spring Harbor Press, New York).

The present invention also enables for the preparation of soluble prion proteins in cultured cells *in vitro.*

In addition, the present invention relates to vectors comprising a nucleic acid according to the invention.

The cloned fusion protein can be inserted into a suitable vector for expression of the gene. A variety of expression vectors may be used for the expression of the fusion protein, including, but not limited to, plasmids, cosmids, phages, phagemids or viruses. Examples include bacteriophages such as lambda derivatives, or plasmids such as pBR322 or pUC plasmids. Typically, such expression vectors comprise a functional origin of replication for propagation of the vector in an appropriate host cell, one or more restriction endonuclease sites for insertion of the prion protein fusion gene sequence into the vector, regulatory elements such as promoters, enhancers and heterologous intron sequences and one or more selection markers for prokaryotic and eukaryotic selection. Expression of the desired fusion protein can be achieved in any host-vector system known to the person skilled in the art.

Another aspect of the present invention relates to host cells, comprising a nucleic acid and/or a vector according to the invention.

Preferred are eukaryotic expression systems such as yeast, insect, mammalian cells and transgenic animals. Preferred mammalian host cells include but are not limited to those derived from human, monkeys and rodents, (see, for example, Kriegler M. in Gene Transfer and Expression: A Laboratory Manual, Freeman & Co, New York, 1990), such as monkey kidney cell line transformed with SV40 (COS-7, American Type Culture Collection (ATCC) CRL 1651), human embryonic kidney cells (293, 293-EBNA), baby hamster kidney cells (BHK, ATCC, CCL10), Chinese hamster ovary cells (CHO). For long term, high yield production of properly processed soluble prion protein, stable expression in mammalian or insect cells is preferred over transient expression.

Purification of the soluble fusion protein can be performed by standard protein purification methods. For example, a number of methods are known that are based on the specific molecular interaction of a tag and its binding partner. Examples for this are protein A or protein G affinity chromatography, a method that is generally applicable to purifying recombinant proteins that are fused to the constant regions of immunoglobulins. Supernatants of transfected cells which contain the secreted product of the recombinant fusion gene can be directly applied to e.g. a protein A column and the bound protein can be eluted by various buffer systems known in the art.

Those hybrid proteins that are resistant to conversion to the PrP^{sc} form are useful for prophylactic and/or therapeutic applications in mammals.
In an additional aspect the present invention therefore also relates to the use of a hybrid protein according to the invention, a nucleic acid encoding said hybrid protein, a vector, and/or a host cell comprising a nucleic acid encoding said hybrid protein for the preparation of a medicament for the prevention and/or treatment of transmissible spongiform encephalopathies (TSEs).

In contrast to anti-PrP antibodies (Heppner et al., 2001), soluble lymphotoxin-β receptor (Montrasio et al., 2000), and other compounds that exert antiprion activity only after peripheral inoculation thereof (Aguzzi et al., 2001a), the hybrid proteins of the invention have already been demonstrated to be efficacious after both intracerebral and intraperitoneal challenge with prions (see the examples below).

For therapy and prophylaxis an effective amount of a hybrid protein according to the invention, a nucleic acid encoding said hybrid protein, a vector, and/or a host cell comprising a nucleic acid encoding said hybrid protein is administered to said mammal.

The term "therapy" or "therapeutic" is intended to refer to all processes, wherein there may be a slowing, interrupting, arresting, or stopping of the progression of a TSE related disease or condition, but does not necessarily indicate a total elimination of all symptoms.

The term "prophylaxis" or "prophylactic" as used herein is to be understood in the context of the inhibition or delay of the onset of one or more of the symptoms of a TSE related disease or condition.

As used herein, the term "mammal" refers to a warm blooded animal. It is understood that guinea pigs, dogs, cats, rats, mice, horses, cattle, sheep, monkeys, chimpanzees and humans are examples of mammals and within the scope of the meaning of the term. The application in humans is preferred.

In effecting treatment or prophylaxis of a mammal in need of TSE treatment or prophylaxis, the above mentioned hybrid proteins, vectors, nucleic acids or host cells can be administered in any form or mode which makes these compounds bioavailable in an effective amount, including oral or parenteral routes. For example, products of the present invention can be administered intraperitoneally, intranasally, buccally, topically, orally, subcutaneously, intramuscularly, intravenously, transdermally, rectally, and the like. Preferred administration routes are subcutaneous, intravenous, intraperitoneal, intramuscular, intradermal or mucosal administration. More preferably, hybrid proteins are administered intravenously, intracerebrally, or intraperitoneally.

For example, one may transduce the nucleic acids according to the invention to mammalian subjects using suitable viral vectors, such as lentiviral or adenoviral vectors. Further, in a DNA-vaccination-like approach, the vectors and/or nucleic acids according to the invention may be injected subcutaneously. Also, the host cells expressing the hybrid proteins according to the invention may be transferred to mammals that are at risk for, or are infected with, prions. As such cells one may take bone marrow, "neo organs" made of fibroblasts and goretex fibers (Moullier et al., 1993), or "ravioli" of encapsulated producer cells (Aebischer et al., 1996).

One skilled in the art of preparing formulations can readily select the proper form and mode of administration depending upon the particular characteristics of the product selected, the disease or condition to be treated, the stage of the disease or condition, and other relevant circumstances. (Remington's Pharmaceutical Sciences, Mack Publishing Co. (1990)). The products of the present invention can be administered alone or in the form of a pharmaceutical preparation in combination with pharmaceutically acceptable carriers or excipients, the proportion and nature of which are determined by the solubility and chemical properties of the product selected, the chosen route of administration, and standard pharmaceutical practice. For oral application suitable preparations are in the form of tablets, pills, capsules, powders, lozenges, sachets, cachets, suspensions, emulsions, solutions, drops, juices, syrups, while for parenteral, topical and inhalative application suitable forms are solutions, suspensions, easily reconstitutable dry preparations as well as sprays. Compounds according to the invention in a sustained-release substance, in dissolved form or in a plaster, optionally with the addition of agents promoting penetration of the skin, are suitable percutaneous application preparations. The products of the present invention, while effective themselves, may be formulated and administered in the form of their pharmaceutically acceptable salts, such as acid addition salts or base addition salts, for purposes of stability, modulation of hydrophobicity, increased solubility, and the like.

The amount of active agent, i.e. hybrid protein, to be administered to the patient depends on the patient's weight, on the type of application, symptoms and the severity of the illness. Normally, 0.1 mg/kg to 25 mg/kg of at least one hybrid protein is administered, but when applied locally, e.g. intracoronary administration, much lower total doses are also possible.

A further aspect of the present invention relates to a pharmaceutical composition comprising a hybrid protein according to the invention, a nucleic acid encoding said hybrid protein, a vector, and/or a host cell comprising a nucleic acid encoding said hybrid protein, and a pharmaceutically acceptable carrier.

Another aspect of the present invention relates to a process for producing a hybrid protein according to the invention comprising the step of culturing a host cell comprising a nucleic acid and/or a vector according to the present invention under conditions that allow for the expression of said hybrid protein.

The present invention also relates to a transgenic animal, preferably a transgenic mouse, stably expressing a hybrid protein according to the invention.

In this respect, the invention provides methods for the generation of transgenic animals expressing *in vivo* soluble hybrid prion proteins. Methods for the generation of transgenic animals expressing a protein are well known to the person skilled in the art. In a specific embodiment, the present invention discloses mice that express a soluble hybrid protein. Said hybrid proteins are not converted into a pathogenic form and are safe for administration to humans and other mammals suffering from TSEs. Importantly, soluble hybrid proteins delay the onset of a prion disease after intracerebral or intraperitoneal prion inoculation. The biological significance of this finding is robust, because (1) the delay of pathogenesis occurs in several independently established lines of transgenic mice, (2) the delay is detectable upon both intracerebral and intraperitoneal challenge, (3) the buildup of PrP^{sc} is delayed in spleen and brain of transgenic mice, and (4) the prion infectivity is reduced in transgenic spleens and brains when compared to wild-type brains at equivalent time points.

In a further aspect the present invention relates to bone marrow of a transgenic animal according to the invention.

In this respect, the invention discloses the therapeutic efficacy of soluble hybrid proteins according to the invention in bone marrow reconstituted mammals. In a further specific embodiment it has been demonstrated for mice that wild-type mice that have been reconstituted with bone marrow from a transgenic animal express said soluble hybrid protein. The successful reconstitution of bone marrow with the compounds of the invention can be validated using ELISA or similar immunodiagnostic techniques known to the person skilled in the art. Soluble hybrid protein leads to a dramatic reduction in the amount of PrP^{sc} in the spleens of reconstituted infected mammals.

Alternatively, genetically modified bone marrow expressing a soluble hybrid protein may be generated by genetically modifying bone marrow using a transducing virus (such as lentivirus or murine stem-cell virus) containing the soluble protein, or by other delivery ways known to the person skilled in the art.

In another aspect the present invention relates to the use of said bone marrow for the preparation of a medicament for the treatment of transmissible spongiform encephalopathies (TSEs).

The present invention also relates to compositions comprising the hybrid protein according to the invention, as well as solid phase materials, e.g. magnetic beads, carrying same.

The hybrid protein according to the invention and/or said compositions and/or carriers are further useful for removing and/or inactivating PrP^{sc} from biological material such as body fluids. Accordingly, the hybrid protein according to the invention may be used to capture PrP^{sc} from fluids used for therapeutic purposes (such as coagulation factors, therapeutic proteins derived from humans or cultured cells exposed to fetal calf serum) in order to render the fluids non-infectious.

Still further the present invention provides a process of removing PrP^{sc} from biological materials.

The present inventors have demonstrated in contrast to anti-PrP antibodies (Heppner et al., 2001), soluble lymphotoxin-β receptor (Montrasio et al., 2000), and other compounds that exert antiprion activity only after peripheral inoculation (Aguzzi et al., 2001 a), that hybrid proteins according to the invention are efficacious after both intracerebral and intraperitoneal challenge with prions. In one particular embodiment expression analysis established that the hybrid protein: PrP^{c} ratio was vastly substoichiometric (<1:12) in brains and spleens (<1:3.8) of one particular line of transgenic mice analyzed, which demonstrates a remarkable antiprion efficacy. The potency of a soluble hybrid protein in contrasting prion disease pathogenesis demonstrate that soluble hybrid proteins of the invention (or of fragments thereof) represent a new class of antiprion agents. The retardation of lymphoreticular prion replication after peripheral exposure to the hybrid proteins shows that soluble PrP is also useful for post-exposure prophylaxis (Aguzzi, 2003). Variations and refinements of the soluble PrP lead, including the engineering of dominant-negative amino acid substitutions (Perrier et al., 2002), are likely to increase its efficiency even further and are specifically encompassed by the scope of the present invention.

**Table 1**

| Determination of prion infectivity titers in brain and spleen of *tg*550^{*} and wild-type mice challenged intracerebrally or intraperitoneally with prions. In all cases, 30 µl of brain or spleen homogenates were injected intracerebrally into tga20 indicator mice. | | | | | | |
|---|---|---|---|---|---|---|
| Primary inoculation | | | | Intracerebral transmission to *tg*a20 mice | | Calculated infectivity |
| Inoculation route | Genotype | dpi | Tissue | Attack rate | Incubation time (mean ± SD) | log LD₅₀/g tissue |
| i.c. | Wild-type | 48 | Brain | 2/2 | 90 ± 0 | 3.5 |
| i.c. | Wild-type | 48 | Brain | 5/5 | 84.8 ± 8.4 | 4 |
| i.c. | *Tg*550⁺ | 48 | Brain | 4/5 | 110.6 ± 23.8^{a} | 1.7 |
| i.c. | *Tg*550⁺ | 48 | Brain | 0/5 | > 180 | < 1.5 |
| i.c. | Wild-type | 102 | Brain | 3/3 | 62.3 ± 1.1 | 6 |
| i.c. | Wild-type | 102 | Brain | 4/4 | 60.3 ± 3.4 | 6.1 |
| i.c. | *Tg*550⁺ | 102 | Brain | 3/3 | 69 ± 3.6 | 5.4 |
| i.c. | *Tg*550⁺ | 102 | Brain | 4/4 | 65.5 ± 5.2 | 5.7 |
| | | | | | | |
| i.p. | Wild-type | 48 | Spleen | 4/4 | 74.75 ± 4.5 | 4.9 |
| i.p. | Wild-type | 48 | Spleen | ¾ | 77,77,77, > 94 | 4.7 |
| i.p. | *Tg*550⁺ | 48 | Spleen | 4/4 | 87.5 ± 1 | 3.7 |
| i.p. | *Tg*550⁺ | 48 | Spleen | ¼ | 87, >100 | <3 |
| i.p. | Wild-type | 48 | Brain | 0/4 | > 120 | < 1.5 |
| i.p. | Wild-type | 48 | Brain | 0/4 | > 120 | < 1.5 |
| i.p. | *Tg*550⁺ | 48 | Brain | 0/4 | > 120 | < 1.5 |
| i.p. | *Tg*550⁺ | 48 | Brain | 0/4 | > 120 | < 1.5 |

| | | | | | | |
|---|---|---|---|---|---|---|
| ^{a} one mouse did not develop disease after >150 dpi | | | | | | |

### Figures

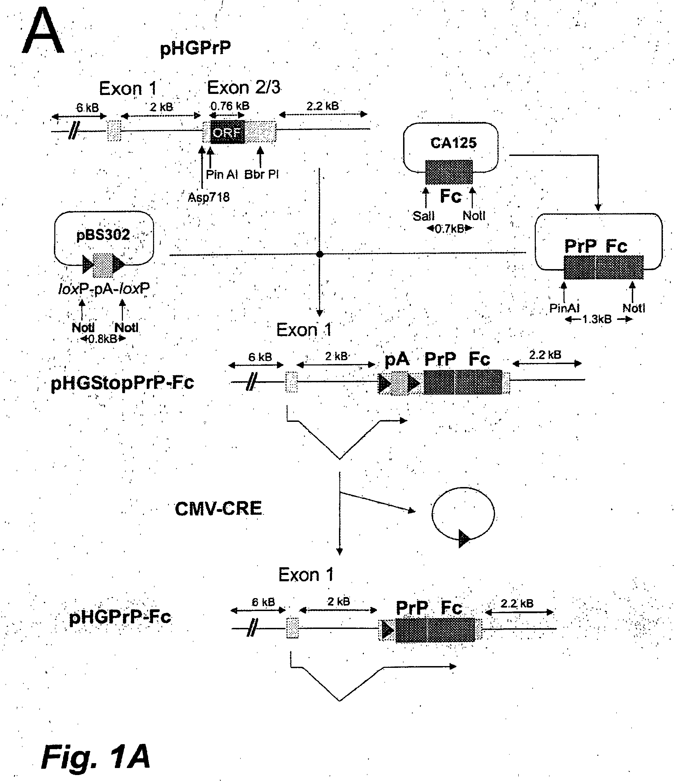

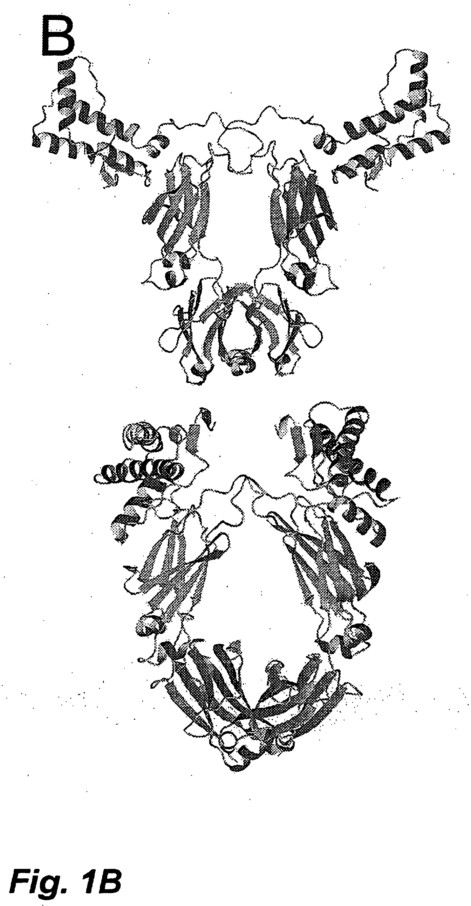

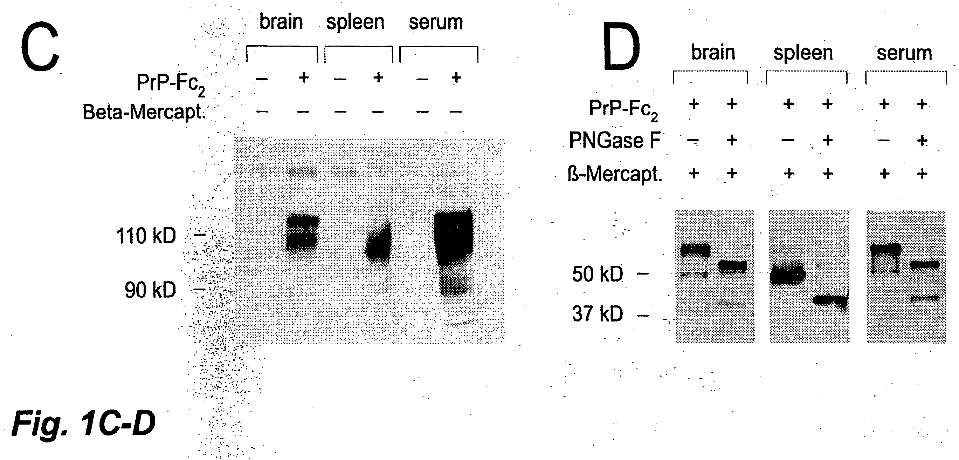

- **Fig. 1**: relates to the generation and characterization of transgenic mice expressing PrP-Fc₂.
**Panel A** shows the cloning strategy for obtaining a conditional expression of soluble PrP. Transgenic mice expressing the half genomic vector (Fischer al, 1996) carrying a stop cassette flanked by IoxP sites and the fusion protein PrP-Fc in exon 2/3 were intercrossed with CMV-Cre deleted mice. This led to the removal of the stop cassette and secretion of soluble PrP in tissues expressing endogenous PrP^{c}.
**Panel B** shows two hypothetical models of PrP-Fc₂ that were created using the NMR and X-ray structures of murine PrP^{c} (121-231) and human Fc_{γ}, connected with the unstructured N-terminus of the latter in an extended conformation. Two different dispositions of PrP^{c} and Fc_{γ} were used to start the simulations that resulted in the two models depicted here.
**Panels C and D** are Western blots of serum, brain and spleen homogenates from wild-type and *tg*550⁺ mice, after immunoprecipitation with the anti-PrP ICSM18, revealed by ICSM18. PrP-Fc₂ is present as a homodimer PrP-Fc₂ in non-denaturating conditions at about 130 kD and (**D**) as a monomer in reducing conditions at about 65 kD in brain and serum. Minor additional smaller bands are probably due to partial degradation of the fusion protein. In spleen, PrP-Fc₂ is present as a dimer at approximately 100 kD in non-reducing conditions (**C**) and as a monomer at about 50 kD in reducing conditions (**D**).

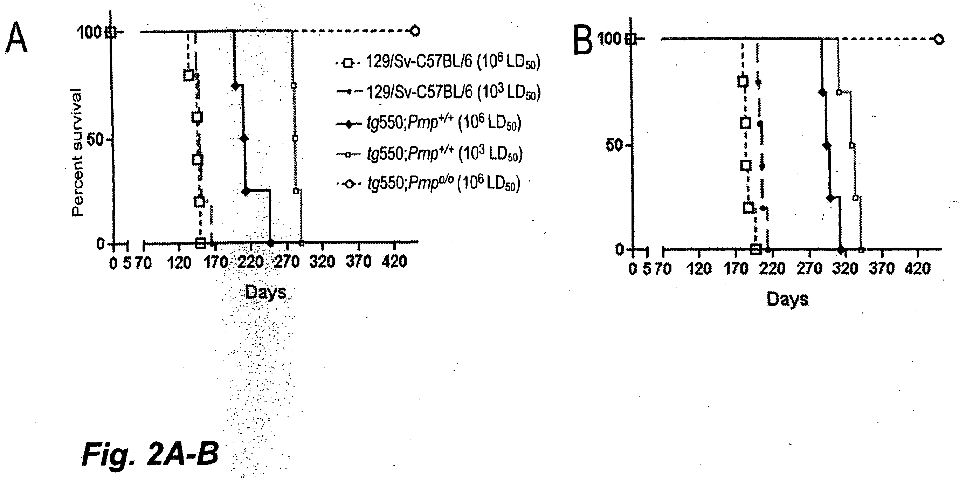

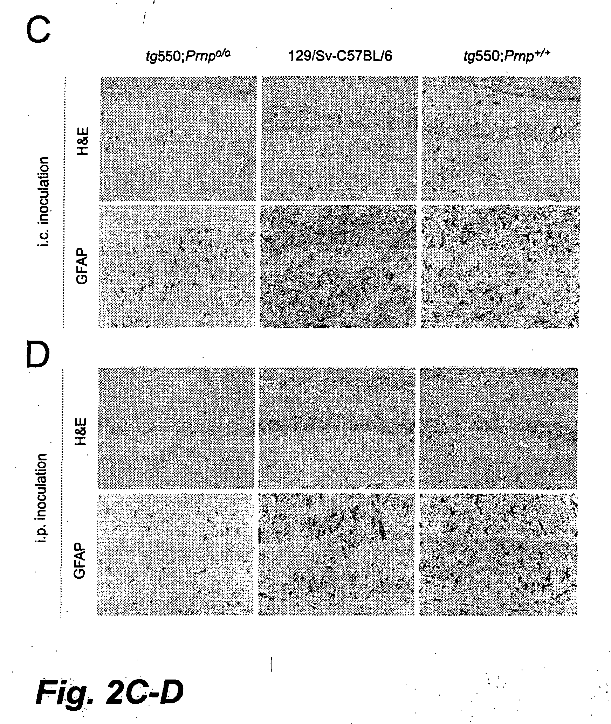

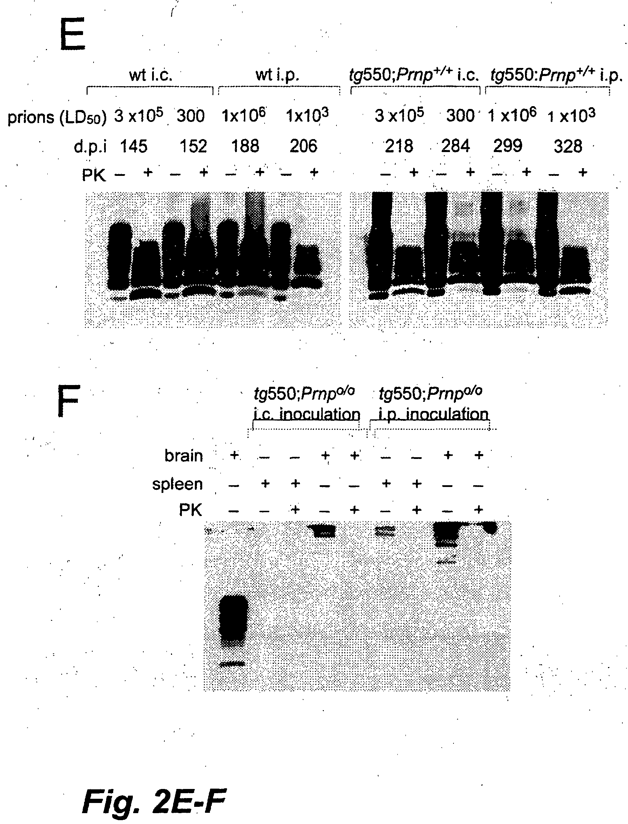

- **Fig. 2**: relates to Scrapie pathogenesis in transgenic mice overexpressing PrP-Fc₂.
**Panels A and B** are survival plots displaying the incubation time (days) until development of terminal scrapie in *tg*550;*Prnp*^{*+l+*} and 129/Sv x C57BU6 control mice after intracerebral **(A)** and intraperitoneal **(B)** challenge of prions. The occurrence of terminal scrapie after both intracerebral and intraperitoneal exposure was strongly delayed in *tg550;Prnp*^{*+l+*} mice, whereas *tg*550;*Prnp*^{*%*} did not develop the disease (>450 days after inoculation).
Panels C and D show that histological analysis of the hippocampal pyramidal cell ribbon upon intracerebral (**C**) and intraperitoneal challenge (**D**) with prions revealed pathological changes in *tg*550;*Prnp*^{+/+} and wild-type mice terminally sick. Mice had diffuse microvacuolation of brain tissue (upper row) and activation of astrocytes with strong expression of GFAP (lower row). Brain tissue of *tg*550;*Prnp*^{*%*} did not reveal pathological changes typical of scrapie, except mild gliosis consistent with ageing.
**Panel E** show that similar quantities and patterns of glycosylated PrP^{sc} were detected in brains of terminal sick *tg*550;*Prnp*^{+/+} mice as in brains from wild-type sick mice.
**Panel F** shows a Western blot analysis of brain and spleen tissues of tg550. *Prnp*^{*%*} sacrificed 450 days after inoculation did not reveal the presence of protease-resistant PrP^{sc}.

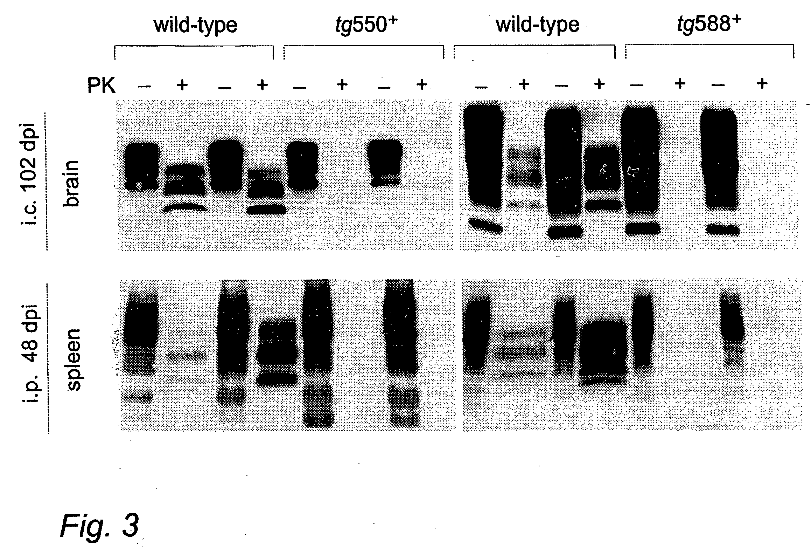

- **Fig. 3**: relates to prion accumulation in transgenic PrP-Fc₂ mice.
Western-blot analysis of prion protein in spleen and brain of mice at indicated dpi, undigested or digested with PK, upon intracerebral and intraperitoneal inoculation. Whereas strong protease-resistant signals of 27-30 kD were visible in all spleens and brains from wild-type animals at all displayed time points, mutant mice showed very faint bands or undetectable signal in brain after intracerebral inoculation at a late time point and in the spleen after intraperitoneal injection at an early time point, meaning that scrapie accumulation is delayed in mice overexpressing soluble PrP in both spleen and brain. Mice were inoculated with 300 IU and 1×10³ IU of scrapie prions with intracerebral and intraperitoneal challenge respectively.

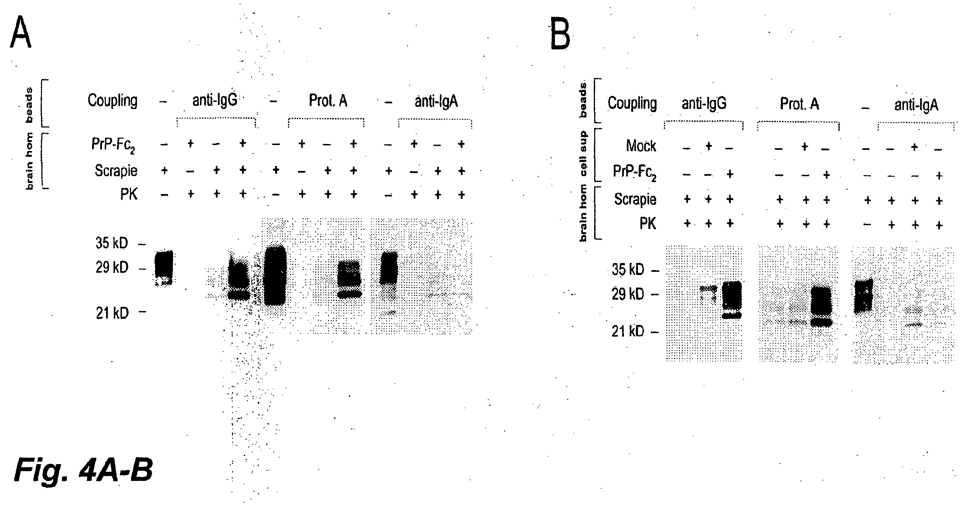

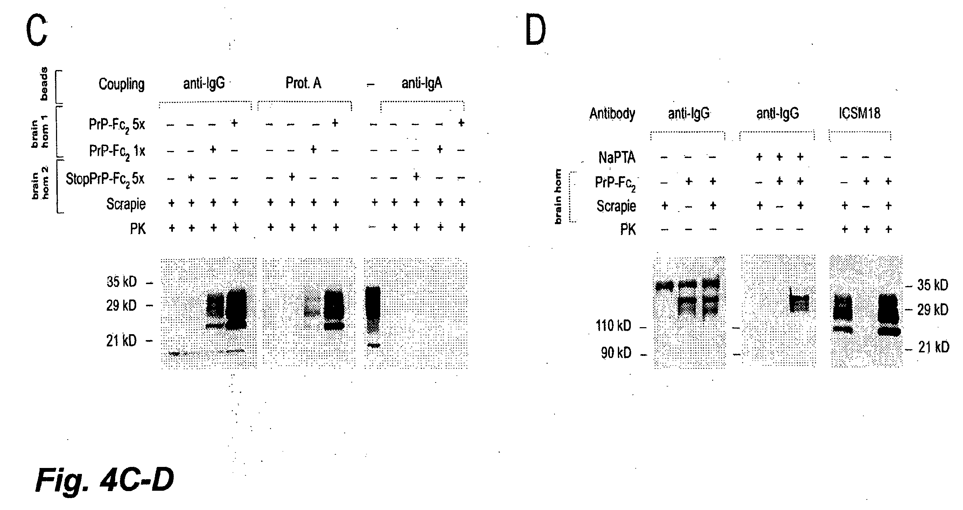

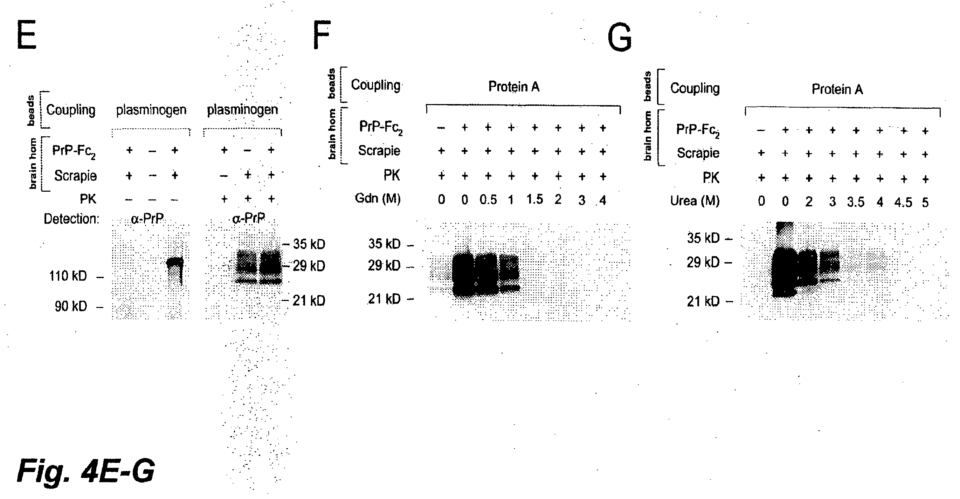

- **Fig. 4**: shows that PrP-Fc₂ interacts and precipitates with disease-associated prion protein *in vivo* and *in vitro. Scrapie-,* brain homogenates of uninfected mice; *Scrapie+,* brain homogenates of terminally scrapie-sick mice; *PrP-Fc*_{*2*}*+,* supernatant of cells transfected with PrP-Fc₂ plasmid; Mock+, supernatant of cells transfected with a mock plasmid.
**Panel A:** After addition of brain homogenates from PrP-Fc₂ terminally sick mice to beads coupled to anti-human IgG or protein A and PK digestion, Western Blot analysis showed the presence of strong bands representative of PrP^{sc}, arguing in favor of an *in vivo* interaction of PrP-Fc₂ and PrP^{sc}. Immunoprecipitation using anti-human IgA beads as a negative control showed weak signals consistent with background.
**Panel B** PrP-Fc₂ precipitates PrP^{sc} *in vitro*. The supernatant of PrP-Fc₂ or of mock transfected cells was preincubated with indicated beads followed by a second incubation with brain homogenates from terminally sick wild-type mice. After PK digestion, Western Blot analysis revealed strong bands representative of PrP^{s}` only when beads were preincubated with PrP-Fc₂ transfected cells supernatant.
**Panel C** Brain homogenates from uninfected *tg*550⁺ and infected wild-type mice were mixed for 30 min at 37°C prior to immunoprecipitation. When increasing the amount of PrP-Fc₂ brain homogenate (PrP-Fc₂ 5×) to a ratio of 5:1 to wild-type infected brain homogenate (500µg: 100µg), we could increase the intensity of the band representative of PrP^{sc} in comparison to the 1:1 ratio (100µg:100µg), suggesting that PrP-Fc₂ is rate limiting in the interaction PrP^{sc}- PrP-Fc₂.
**Panel D** After NaPTA precipitation, PrP-Fc₂ was found associated in the pellet with PrP^{sc} when using brain homogenate from infected mutant mice, whereas in non infected brain, PrP-Fc₂ was not pelleted.
**Panel E:** Plasminogen beads were incubated with brain homogenates of scrapiesick wild-type and PrP-Fc₂ transgenic brains, as well as uninfected PrP-Fc₂ brains. Prior to the binding assay, homogenates were optionally proteasedigested. Blotted eluates of scrapie-sick wild-type and PrP-Fc₂ brains, but not of healthy PrP-Fc₂ brains, were found to contain PrP^{sc}, whereas anti-Fc antibodies visualized plasminogen-precipitated material only in non-digested scrapie-sick PrP-Fc₂ eluates.
**Panel F** shows the characterization of the PrP^{sc} binding activity of PrP-Fc₂. Brain homogenates from wild-type infected mice were pre-incubated with increasing guanidinium concentrations during 18 hours and then diluted to a concentration shown not to disturb the immunoprecipitation. PrP-Fc₂ binding activity to PrP^{sc} was detectable in presence of 1.5 M guanidinium, but was abolished starting from 2 M guanidinium.
**Panel G:** After denaturation with ≥ 4.5 M urea, no PrP^{sc} could be recovered by precipitation with native PrP-Fc₂. Therefore, interaction of PrP^{sc} with PrP-Fc₂ is dependent on the native conformation of PrP^{sc}. *hom:* homogenate; sup: supernatant; *Gdn:* guanidinium hydrochloride.

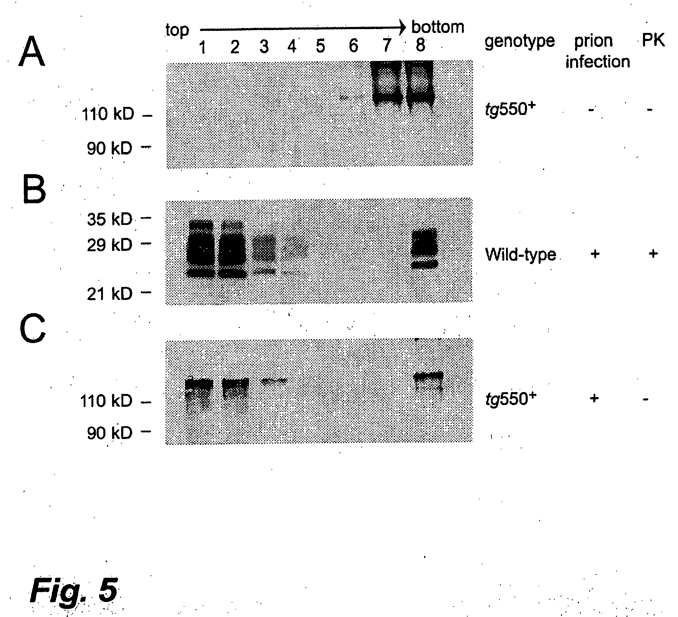

- **Fig. 5**: relates to a flotation assay of brain homogenates.
Brain homogenates from (**A**) healthy *tg*550⁺ mice, (**B**) scrapie-infected mice after PK digestion, and (**C**) infected PrP-Fc₂ animals were prepared in 1% Triton X-100 at 4°C and subjected to a flotation assay. The samples from the Nycodenz density gradient were collected and immunoblotted with anti-PrP ICSM18. In uninfected mice, soluble PrP-Fc₂ is present at the bottom of the gradient (**A**), whereas in infected mice, PrP-Fc₂ (**C**) is found in the top fractions, where lipid rafts are located, in the same fractions as PrP^{sc} (**B**).

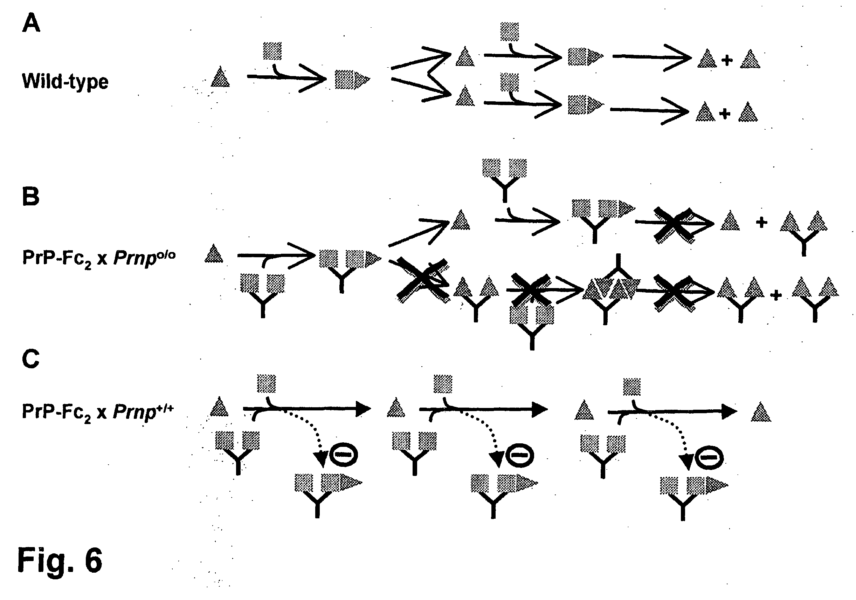

- **Fig. 6**: shows a model for the antiprion action of PrP-Fc₂.
**Panel A** shows the template refolding model of prion replication (upper panel) postulating a transient dimerization of PrP^{c} and PrP^{sc}. As a result, PrP^{sc} imparts its own beta-sheet-rich, protease-resistant conformation onto PrP^{c}. Technical obstacles, however, have thus far prevented direct visualization of a PrP^{c}-PrP^{sc}, complex in vivo.
**Panel B** shows that in the absence of PrP^{c}, soluble dimeric PrP does not support replication of the infectious agent, nor formation of a protease-resistant moiety. Although several lines of evidence indicate that it can associate with PrP^{sc}, this association is non-productive.
**Panel C** shows how mice co-expressing PrP^{c} and soluble dimeric PrP replicate prions and eventually develop scrapie. However, the kinetics with which scrapie pathology develops, prion infectivity replicates, and PrP^{sc} accumulates, is slower than in wild-type mice. All available experimental evidence suggests that PrP-Fc₂ sequesters incoming as well as nascent PrP^{sc}, and renders it unavailable for further template-directed conversion of PrP^{c}.

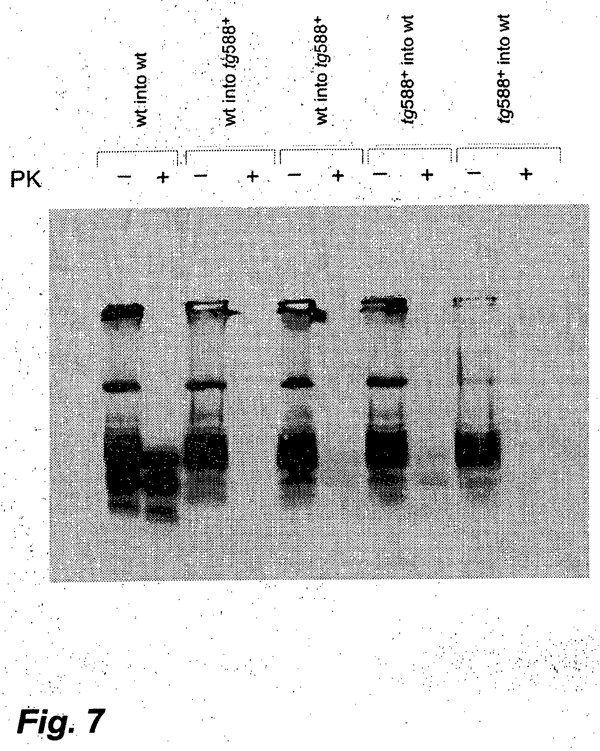

- **Fig.7**: Reduced PrP^{sc} accumulation in prion infected bone marrow reconstituted mice. Western-blot analysis of prion protein in spleen of bone marrow reconstituted mice at 50 dpi, undigested or digested with PK, upon intraperitoneal inoculation. Whereas strong protease-resistant signals of 27-30 kD were visible in the spleens from wild-type animals reconstituted with wild-type bone marrow, all other groups of mice which were shown by ELISA to express PrP-Fc₂ in the serum showed very faint bands or undetectable PrP signals in the spleen meaning that scrapie accumulation is delayed in mice expressing soluble PrP in blood. Mice were inoculated with 1×10³ IU of scrapie prions with intraperitoneal challenge.

### Examples

The following examples further illustrate the best mode contemplated by the inventors in carrying out their invention.

### Example 1 Expressing of soluble dimeric PrP-Fc₂ in transgenic mice

The plasmid CA125 (a kind gift from C. Ambrose, Cambridge) containing a modified IgG₁ open reading frame mutated at the Fe_{γ} receptor and complement binding sites (CH2 domain; L234A, L235E, G237A, and P331S) (Canfield and Morrison, 1991; Lund et al., 1991; Tao et al., 1993) was cut with *Sal*l and *Not*l*,* blunted, and inserted into pJE14/PrP. The resulting plasmid pJE14/PrP-Fc₂ was cut with *PinAl* and *Not*l, and inserted into the fused exon 2/3 of the pPrPHG vector (Fischer et al., 1996) thereby replacing the PrP coding region with the murine PrP (lacking the carboxy terminal GPI attachment signal) fused to the Fc portion of a human lgG1 heavy chain. The binding sites for Fc_{γ} receptors and for complement had been inactivated by mutagenesis (Ettinger et al., 1996): this can be helpful since various components of the complement system are important for efficient prion pathogenesis and their binding to PrP-Fc₂ might exert unpredictable effects on prion pathogenesis. To allow for conditional and tissue-specific expression of the transgene, a loxP-flanked transcriptional termination cassette was inserted into exon 2/3, immediately upstream of the open reading frame (Fig. 1A). A transcriptional stop cassette flanked by two *loxP* sites was inserted into exon 2 of this vector. The plasmid was verified by sequencing, and the insert was excised with *Not*l and *Sal*l. Nuclear injections into fertilized oocytes derived from *Prnp*^{*%*} mice (129/Sv x C57BU6 hybrid background) were performed by conventional methods. Transgene positive offspring were identified by PCR using the primers 602 (5'-CAG AAC TGA ACC ATT TCA ACC GAG) and 603 (5'-TCA GTC CAC ATA GTC ACA AAG AGG G), and by Southern blot analysis of tail DNA. Six founder mice were generated and denominated *Prnp*^{*tm1*}*- Tg(PrP*/*FcStop)Zbz,* from which two lines, *tg*550 (low expressor) and *tg*588 (higher expressor), were bred with *Prnp*^{*%*} (mixed 129/Sv x C57BU6) or with *Prnp*^{+/+} mice (C57BU6). Transgenic mice were then bred to CMV-Cre deleted mice (Schwenk et al., 1995) in order to remove the transcriptional stop cassette from their germ line. Non-floxed mice (*tg*550stop and *tg*588stop) served as controls.

Expression of PrP-Fc₂ in transgenic mice was assessed by quantitative Western blotting analysis. Serial 1:2 dilutions of brain or spleen homogenate in *Prnp*^{*%*} homogenate were electrophoresed through 12.5% SDS-PAGE gels, blotted, probed with antibody ICSM18 (Heppner et al., 2001), and detected by enhanced chemiluminescence. Signals were quantified with a Kodak ImageStation. The PrP-Fc₂:PrP^{c} expression ratio was assessed by determining the homogenate dilution at which the signals for PrP-Fc₂ and for PrP^{c} were of equal intensities. Serum PrP-Fc₂ concentration was measured by ELISA: microtiter plates were coated with ICSM18 (1:1500) and incubated with various dilutions (1:50 to 1:2000) of serum derived from *tg*550 and tg588. Signals were visualized with horseradish peroxidase-coupled goat IgG against human (Rockland) and ABTS. Hypothetical models of PrP-Fc₂ were created using the NMR and X-ray structures murine PrP^{c} (121-231) and scFc connected with the unstructured N-terminus of the latter in an extended conformation. Then, the N-terminus of the scFc was relaxed by performing molecular dynamics simulations with the CHARMM (Brooks et al., 1983) program. Simulations were carried out at 300 K for 1 ns with an implicit treatment of the solvent. We predicted that the PrP-Fc₂ molecule would be assembled as a disulfide-bridged dimer (Dwyer et al., 1999) with two PrP moieties facing each other in an arrangement that resembles the Fab portions of immunoglobulins (Fig. 1B). Under non-reducing conditions, PrP-Fc₂ was detected as a 100-130 kDa protein in serum, brain, and spleen protein extracts of transgenic animals (Fig. 1 C). Under reducing conditions (β-mercaptoethanol), antibodies to PrP and to human Fcγ₁ detected bands of 50-65 kDa in transgenic brains (Fig. 1D). This is half the size of the bands seen under non-reducing conditions. Therefore, PrP-Fc₂ exists as a dimer *in vivo.*

PNGase F digestion, which removes glycans, shifted the reduced PrP-Fc₂ band to a major moiety with the expected molecular weight of 55 kDa, and to a minor band of 40 kDa. The minor band was not detected by an anti-N-terminal PrP antibody (data not shown), suggesting that it represents the N-terminal product of a PrP-Fc₂ cleavage event.

Expression of PrP-Fc₂ was compared to endogenous PrP^{c} in brain and in spleen by quantitative Western blotting. Serial two-fold dilutions of appropriate tissue homogenates were blotted, and the relative expression levels of the two molecules were calculated from calibration curves obtained by acquisition of chemiluminescence, using a method described previously (Heppner et al., 2001). Brain PrP-Fc₂ was expressed approximately 12 and 10-fold less than PrP^{c} in brain of tg550 and tg588, respectively (data not shown). In spleen, PrP-Fc₂ expression was found to be 4-fold less than PrP^{c} in *tg*550 but 1.5-fold more in *tg*588. Therefore, PrP-Fc₂ expression in the brain of *tg*550 and *tg*588 was 8% and 10% that of wild-type animals. In spleen, it corresponded to 27% and 150% of PrP^{c} expression (data not shown).

### Example 2 Infection of transgenic mice expressing a soluble dimeric prion protein. Soluble PrP is not converted into an disease specific PrP form

To investigate whether PrP-Fc₂ can be converted into a self-propagating "PrP-Fc^{sc}" form, the PrP-Fc₂ transgene was bred in *PrnP*^{*%*} mice (Büeler et al., 1992) which are resistant to scrapie (Büeler et al., 1993; Sailer et al., 1994). Adult *tg550-Prnp*^{*%*} mice (*tg*550° for short), which express PrP-Fc₂ but lack endogenous PrP^{c}, were inoculated with RML prions intracerebrally (i.c.) or intraperitoneally (i.p.) with 30µl and 100µl, respectively, of brain homogenate containing 3×10² to 10⁶ LD₅₀ infectious units of Rocky Mountain Laboratory strain (RML, passage 5.0) scrapie prions prepared as described previously (Klein et al., 1997). In order to uncover possible dose-dependent effects, saturating as well as rate-limiting doses of prions were administered.

Tg550° mice were healthy at >450 days post inoculation (dpi) by the i.p or i.c. route, whereas all *Prnp*^{+/+} control mice (129/Sv × C57BU6) succumbed to scrapie at ≤ 206 dpi (Fig. 2A-B). Inoculated transgenic animals were sacrificed, and brain and spleen protein extracts subjected to immunoblot analysis. No traces of protease-resistant prion protein were detected in brains and spleens after PK digestion (Fig. 2F), even when PrP^{sc} was concentrated by sodium phosphotungstate (NaPTA) precipitation (Heppner et al., 2001; Safar et al., 1998) which allows for detection of brain PrP^{sc} at concentrations 10⁴-10⁵ fold lower than those typically reported in terminally sick brains. Prion replication in scrapie-inoculated *tg*550° mice was assessed by i.c. inoculation of 3 mg brain homogenate (taken at 450 dpi) to four tga20 indicator mice. None of the indicator mice developed scrapie at ≥ 150 dpi, whereas as little as 3×10⁻⁸ g brain homogenate from terminally sick *Prnp*^{+/+} mice induced scrapie in 4/4 *tg*a20 mice.

For histological analysis, organs were fixed in 4% paraformaldehyde in PBS, paraffin-embedded, cut into 2µm sections and stained with hematoxylin/eosin (HE). Immunostaining for glial fibrillary acidic protein (GFAP) was performed using a rabbit-anti-GFAP antiserum (DAKO, 1:300) and visualized with biotinylated swine-anti-rabbit serum (DAKO, 1:250), avidin-peroxidase (DAKO) and diaminobenzidine (Sigma). Histological analysis failed to reveal any vacuolation on brain sections from inoculated *tg*550° mice following HE staining (Fig. 2C-D). Immunohistochemistry for the astrocytic activation marker, glial fibrillary acidic protein, revealed minimal astrogliosis consistent with ageing (Fig. 2C-D). All of the above results firmly establish that PrP-Fc₂ does not support prion replication and is not transformed into a protease-resistant protein after prion infection.

### Example 3 PrP-Fc₂ antagonizes PrP^{sc} accumulation in brain and spleen of transgenic mice

*tg*550⁺, *tg*588⁺*,* and their wild-type littermates were inoculated with a low, rate-limiting dose of prions intracerebrally or intraperitoneally. Deposition of PrP^{sc} was analyzed by NaPTA-enhanced Western blotting (Safar et al., 1998). Phosphotungstic acid (NaPTA) precipitation was performed as described (Heppner et al., 2001; Safar et al., 1998) with 500 µg or 1 mg of 10% tissue homogenate (brain and spleen). Precipitates were resuspended in 0.1% Sarcosyl. NaPTA precipitates of brain and homogenates were run on SDS-PAGE (5% stacking and 8, 12 or 16% resolving) and transferred on nitrocellulose (Schleicher & Schuell) by wet blotting. Membranes were blocked with 5% Top-Block (Juro) in Tris-buffered saline-Tween (TBS-T) at room temperature (RT) for 1 hour, incubated in 1% Top-Block in TBS-T with ICSM18 (for PrP) and PrP-Fc₂ detection or an goat-anti-human IgG (Rockland) (for PrP-Fc₂) 1 hour at RT or overnight at 4°C, washed three times for 15 min in TBS-T, incubated with 1% Top-Block in TBS-T and rabbit-anti-mouse IgG₁-horseradish peroxidase (Zymed, 1:10,000) in case of ICSM18, washed three times for 15 minutes in TBS-T, and developed using enhanced chemiluminescence (ECL) detection reagents. PrP^{sc} was analyzed in brains of intracerebrally inoculated mice at 102 dpi (when buildup of prion infectivity is increasing exponentially), and in spleens of i.p. inoculated mice at 48 dpi (a time point at which infectivity and PrP^{sc} should have reached a plateau phase). PrP^{sc} was readily detected at these time points in wild-type brains and spleens, whereas no PrP^{sc}, or just traces thereof, were present in *tg*550⁺ and *tg*588⁺ organs (Fig. 3). Therefore, PrP-Fc₂ inhibits PrP^{sc} accumulation in both central nervous system and spleen.

In parallel, prion titers in brains and spleens were determined at 48 and 102 days after inoculation by bioassay with *tg*a20 mice (Fischer et al., 1996). Whereas brains of wild-type mice contained sizeable prion titers of 3.5-4 log LD₅₀/g tissue at 48 days after intracerebral inoculation, infectivity in *tg*550⁺ brains was undetectable or barely detectable. At 102 dpi, however, prion titers in *tg*550⁺ brains were about to catch up, being only 0.5 log₁₀ units less than wild-type controls (Table 1).

Following intraperitoneal inoculation, prions colonize the lymphoreticular system by a complex process which is dependent on mature follicular dendritic cells (FDCs) (Montrasio et al., 2000) and eventually results in neuroinvasion. It was found that this process is also antagonized by PrP-Fc₂. Prion titers in spleens of wild-type mice harvested at 48 dpi after intraperitoneal inoculation reached 4.7-4.9 logLD₅₀ units/g, which corresponds to the maximal plateau levels that are typically reached in lymphoreticular organs. Instead, prion infectivity in the spleen of one *tg*550⁺ mouse was 1 log lower (3.7 logLD₅₀ units/g), whereas a second *tg*550⁺ spleen contained barely detectable infectivity and induced scrapie in tga20 indicator mice with an attack rate of only ¼ (Table 1). This indicates that PrP-Fc₂ not only impairs prion replication within the central nervous system, but also in lymphoreticular organs. It was formally excluded that lymphoreticular slow-down of prion replication may be due to impaired "wash-back" of brain infectivity, since no prions were detectable in brains of intraperitoneally inoculated mice of either genotype at 48dpi - indicating that prions had not yet reached the central nervous system at this early time point.

### Example 4 PrP-Fc₂ binds to PrP^{sc} in vivo and in vitro

In a first experiment, tosyl-activated paramagnetic beads were coupled covalently with (1) antibodies to human Fc_{γ1}, (2) protein A, or (3) antibodies to human IgA. The two former reagents were expected to bind the Fc_{γ} portion of PrP-Fc₂, while the third served as a negative control. When incubated with native brain homogenate of scrapie-sick PrP-Fc₂ mice, reagents 1 and 2 (but not reagent 3) precipitated a protein complex that contained protease-resistant PrP^{sc} (Fig. 4A). Instead, only background levels of PrP^{sc} were captured from scrapie-sick wild-type mice. Therefore, surface-immobilized reagents to human Fc_{γ1} precipitate multiprotein complexes that contain protease-resistant PrP. This provides a first line of evidence that PrP^{sc} and PrP-Fc₂ interact in vivo.

### Surface-immobilized PrP-Fc₂ efficiently captures PrP^{sc} in pull-down assays

To trap PrP^{sc} with PrP-Fc₂, bound to a solid-phase matrix in vitro paramagnetic beads were coupled (1) with mouse-anti-human-IgG, (2) with protein A, or (3) with mouse-anti-human-IgA. Beads were incubated with the supernatant of COS-7 cells transfected with a PrP-Fc₂ expression construct, washed, and incubated with prion-infected wild-type brain homogenate. The precipitate was digested with PK, blotted and probed with antibodies to PrP or to human IgG.

Immobilized PrP-Fc₂ readily captured PrP^{sc} from scrapie-sick wild-type brains, whereas no PrP^{sc} was recovered with beads that had been pre-incubated with mock-transfected cell supernatant (Fig. 4B). Efficient capture was obtained also with brain homogenate that had been subjected to digestion with proteinase K prior to immunoprecipitation, indicating that PrP-Fc₂ binds PrP²⁷⁻³⁰ in addition to PrP^{sc} (data not shown). Interaction was specific and required the presence of both PrP-Fc₂ and PrP^{sc}, as no PrP was captured if recombinant PrP-Fc₂ was omitted, nor with beads covalently linked to anti-IgA antibodies which do not recognize human Fc_{γ}.

### PrP-Fc₂ interacts with PrP^{sc} in liquid phase

Capture of PrP^{sc} by affinity-purified PrP-Fc₂ in a highly sensitive pull-down assay may identify weak interactions that are not detectable in vivo: a number of serum proteins including fibrinogen, antithrombin III, and factor IX can immobilize protease-digested PrP^{sc} in vitro (Fischer et al., 2000), but may not necessarily interact with PrP^{sc} in living cells. This may be because (1) direct covalent linking to paramagnetic beads may partially denature the bait protein and expose hydrophobic patches that interact with PrP^{sc} nonspecifically; (2) because affinity capture of PrP-Fc₂ by anti-IgG or protein A beads may result in highly repetitive, anisotropic arrays of very high avidity; or (3) the local concentration of the bait on the surface of the beads may be much higher than that attainable in vivo.

To identify interactions between PrP^{sc} and PrP-Fc₂ in the liquid phase it was investigated whether capture would occur also with concentrations of PrP-Fc₂ present in transgenic brains.
Equal amounts (100:100 µg) of brain extracts from scrapie-sick wild-type mice and from uninfected PrP-Fc₂ mice were co-incubated. Subsequent capture with anti-human-IgG and protein A beads led to the recovery or PrP^{sc} from this brain homogenate mixture, providing further evidence that binding of PrP-Fc₂ and PrP^{sc} can occur *in vitro* (Fig. 4C). Instead, when scrapie-sick wild-type brain extract was mixed with up to 500 µg of uninfected *StopPrP-Fc* brain homogenate, no PrP^{sc} was detected, confirming the specificity of our assay system (Fig. 4C). Mouse-anti-human-IgA coupled beads were used as additional control (Fig. 4A-C). In the latter experiments no bands were ever detected with the electrophoretic motility characteristic of PrP^{sc}, or of PrP-Fc₂.

There was an obvious increase in precipitated PrP^{sc} when the amount of co-incubated uninfected PrP-Fc₂ homogenate was increased fivefold (100:500 µg), suggesting that availability of PrP-Fc₂ is rate-limiting. The latter observation is in line with the fact that the ratio of PrP-Fc₂ to PrP^{c} is vastly substoichiometric (data not shown).

### Upon prion infection, PrP-Fc₂ becomes precipitable by NaPTA and by plasminogen.

It was then investigated whether, upon prion infection, PrP-Fc₂ would acquire some of the biophysical properties of PrP^{sc}. A remarkable property of PrP^{sc} is that it readily sediments in the presence of 4% sodium phosphotungstate (NaPTA), whereas PrP^{c} remains soluble (Safar et al., 1998). Accordingly, NaPTA precipitations of healthy and scrapie-sick *tg*550⁺ and wild-type brain homogenates were performed. Pellets and supernatants were then optionally protease-digested, blotted, and probed with antibodies to human Fcγ or to PrP. As expected, NaPTA precipitates of both wild-type and transgenic scrapie-sick mice contained protease-resistant PrP^{sc}. Moreover, NaPTA pellets from terminally-sick (but not from uninfected) *tg*550⁺ yielded strong signals with anti-Fcγ antibodies (Fig. 4D). Therefore, PrP-Fc₂, like PrP^{sc}, becomes NaPTA-precipitable upon infection of tissue co-expressing PrP-Fc₂ and PrP^{c}. This finding demonstrates that PrP-Fc₂ and PrP^{sc} form a complex in vivo. Moreover, proteolytic digestion of the precipitate yielded only moieties with the molecular weight of PrP²⁷⁻³⁰, indicating that PrP-Fc₂ does not become protease-resistant as a consequence of complexing with PrP^{sc}.

In the presence of high concentrations of detergents, surface-immobilized plasminogen can also be used to differentiate between PrP^{c} and PrP^{sc} (Fischer et al., 2000). It was therefore tested whether PrP-Fc₂ would bind plasminogen. Tosyl-activated paramagnetic beads were covalently linked to human plasminogen, and incubated with homogenates of scrapie-sick or uninfected wild-type or *tg*550⁺ brains. Prior to the binding assay, homogenates were optionally protease-digested. Blotted eluates of scrapie-sick wild-type and *tg*550⁺ brains, but not of healthy brains, were found to contain PrP^{sc} (Fig. 4E), whereas anti-Fc antibodies visualized plasminogen-precipitated material only in non-digested scrapie-sick *tg*550⁺ eluates (Fig. 4E, left panel). Therefore, after infection of mice co-expressing PrP^{c} and PrP-Fc₂, the latter becomes precipitable by plasminogen. This finding provides further evidence that PrP-Fc₂ and PrP^{sc} form a complex *in vivo.*

### Denaturation of PrP^{sc} abolishes its capability to interact with PrP-Fc₂

It was then determined whether binding of native PrP-Fc₂ is dependent on the conformation of PrP^{sc}. Brain homogenate from terminally sick wild-type mice was denatured by preincubation for 18 hours in increasing concentrations of guanidinium hydrochloride (GdnHCI) or of urea, diluted 50-fold in order to reduce the concentration of chaotropic salts below the denaturation threshold, and incubated with uninfected *tg*550⁺ brain homogenate (100µg:500µg). Proteins were then captured with protein A beads, digested with PK, and blotted. After denaturation with ≥ 1.5M guanidinium hydrochloride (Gdn, Fig. 4F) or with ≥ 4.5 M urea (Fig. 4G), no PrP^{sc} could be recovered by precipitation with native PrP-Fc₂. Therefore, interaction of PrP^{sc} with PrP-Fc₂ is dependent on the native conformation of PrP^{sc}. Interestingly, the concentration of Gdn or urea sufficient to abrogate binding appears to be much lower than that needed to sterilize prion infectivity.

### Upon prion infection, PrP-Fc₂ relocates to lipid rafts.

Then an independent, completely unrelated experimental approach was used to confirm the interaction between PrP^{sc} and PrP-Fc₂ *in vivo.* Like other glycosyl phosphatidyl inositol (GPI) anchored proteins, PrP^{sc} and is inserted into cholesterol rich membrane micro-domains denominated lipid rafts (Naslavsky et al., 1997). Following extraction in cold Triton X-100 and ultracentrifugation on density gradients, proteins associated with lipid rafts float in the uppermost fractions.

When prepared in cold TX-100 and subjected to a "flotation assay" (ultracentrifugation in a Nycodenz gradient), it was found that PrP^{sc} floated with the lower-density fractions of the gradient (Fig. 5B), which confirms earlier reports (Naslavsky et al., 1997). Instead PrP-Fc₂, which is not expected to associate with lipids, migrated with the higher-density fractions (Fig. 5A). In contrast, prion infection shifted the buoyancy of Fcγ-positive PrP-Fc₂ from *tg*550⁺ brains towards the upper fractions of the gradient, as would be expected from a resident of rafts (Fig. 5C). This finding shows that the subcellular localization of PrP-Fc₂ in transgenic mice experiences a dramatic shift in vivo as a consequence of prion infection, and demonstrates that secreted PrP-Fc₂ engages in a complex with PrP^{sc} within lipid rafts. Some PrP^{sc} and PrP-Fc₂ were always present at the bottom of the tube in addition to the low-density fractions. This is probably because partial disruption of rafts may detach some proteins and allow for their sedimentation through the density gradient (Fig. 5B-C).

### Example 5 Inhibition of PrPSc synthesis in wild-type mice reconstituted with bone marrow from transgenic PrP-Fc₂ mice

The experiments in example 3 have demonstrated that PrP-Fc₂ when expressed in transgenic mice under control of the PrP promoter not only impairs prion replication within the central nervous system, but also in lymphoreticular organs. In these experiments PrP-Fc₂ was present in all organs where PrP is normally expressed, particularly in nervous and lymphoid tissues. To assess the therapeutic efficacy of soluble prion proteins in TSEs in which the therapeutic agent, PrP-Fc₂, would be administered intravenously or intraperitoneally bone marrow chimeras expressing soluble PrP in the blood were generated. Mice were lethally irradiated and reconstituted with fetal liver cells from donor animals. Briefly, tg588 bone marrow was reconstituted into wild-type mice (1), wild-type bone marrow into tg588 mice (2), wild-type into wild-type (3) and tg588 into tg588 (4). To check if the reconstitution was successful, an ELISA of PrP-Fc₂ from the serum of these mice was performed and showed high levels of soluble PrP-Fc₂ in all mice except in the group of wild-type bone marrow into wild-type mice. (1): OD = 1.2; (2): OD = 2.8; (3): OD = 0.2 and (4) OD = 2.8. The reconstituted mice were inoculated i.p. with a low dose of prions and some mice of all groups except from group 4 were killed 50 days after inoculation (a time point at which infectivity and PrP^{sc} should have reached a plateau phase). PrP^{sc} was readily detected at these time points in spleens of wild-type mice reconstituted with wild-type bone marrow, whereas no PrP^{sc}, or just traces thereof, were present in spleens of mice of the other groups (Fig. 7). Therefore, soluble dimeric PrP-Fc₂ inhibits PrP^{sc} accumulation in the spleen even when expressed only in the lymphoid compartment.

### REFERENCES

Aebischer P, Schluep M, Deglon N, Joseph JM, Hirt L, Heyd B, Goddard M, Hammang JP, Zurn AD, Kato AC, Regli F, Baetge EE. (1996). Intrathecal delivery of CNTF using encapsulated genetically modified xenogeneic cells in amyotrophic lateral sclerosis patients. Nat Med 2(6): 696-9.
Aguzzi, A. (2003). Prions and the immune system: a journey through gut, spleen, and nerves. Adv Immunol *in* press.
Aguzzi, A., Glatzel, M., Montrasio, F., Prinz, M., and Heppner, F. L. (2001a). Interventional strategies against prion diseases. Nat Rev Neurosci 2, 745-749.
Aguzzi, A., Montrasio, F., and Kaeser, P. S. (2001 b). Prions: health scare and biological challenge. Nat Rev Mol Cell Biol 2, 118-126.
Bessen, R. A., Kocisko, D. A., Raymond, G. J., Nandan, S., Lansbury, P. T., and Caughey, B. (1995). Non-genetic propagation of strain-specific properties of scrapie prion protein. Nature 375, 698-700.
Briesewitz R, Ray GT, Wandless TJ, Crabtree GR. (1999). Affinity modulation of small-molecule ligands by borrowing endogenous protein surfaces. Proc Natl Acad Sci USA 96(5):1953-8.
Brooks, B. R., Bruccoleri, R. E., Olafson, B. D., States, D. J., Swaminathan, S., and Karplus, M. (1983). CHARMM: A Program for Macromolecular Energy, Minimization, and Dynamics Calculations. J Comp Chem 4, 187-217.
Büeler, H. R., Aguzzi, A., Sailer, A., Greiner, R. A., Autenried, P., Aguet, M., and Weissmann, C. (1993). Mice devoid of PrP are resistant to scrapie. Cell 73, 1339-1347.
Büeler, H. R., Fischer, M., Lang, Y., Bluethmann, H., Lipp, H. P., DeArmond, S. J., Prusiner, S. B., Aguet, M., and Weissmann, C. (1992). Normal development and behaviour of mice lacking the neuronal cell-surface PrP protein. Nature 356, 577-582.
Canfield, S. M., and Morrison, S. L. (1991). The binding affinity of human IgG for its high affinity Fc receptor is determined by multiple amino acids in the CH2 domain and is modulated by the hinge region. J Exp Med 173, 1483-1491.
DebBurman, S. K., Raymond, G. J., Caughey, B., and Lindquist, S. (1997). Chaperonesupervised conversion of prion protein to its protease- resistant form. Proc Natl Acad Sci U S A 94, 13938-13943.
Dwyer, M. A., Huang, A. J., Pan, C. Q., and Lazarus, R. A. (1999). Expression and characterization of a DNase I-Fc fusion enzyme. : J Biol Chem 274, 9738-9743.
Eigen, M. (1996). Pnonics or the kinetic basis of prion diseases. Biophys Chem 63, A1-18.
Ettinger, R., Browning, J. L., Michie, S. A., van Ewijk, W., and McDevitt, H. O. (1996). Disrupted splenic architecture, but normal lymph node development in mice expressing a soluble lymphotoxin-beta receptor-IgG1 fusion protein. Proc Natl Acad Sci U S A 93, 13102-13107.
Fischer, M., Rülicke, T., Raeber, A., Sailer, A., Moser, M., Oesch, B., Brandner, S., Aguzzi, A., and Weissmann, C. (1996). Prion protein (PrP) with amino-proximal deletions restoring susceptibility of PrP knockout mice to scrapie. EMBO J 15, 1255-1264.
Fischer, M. B., Roeckl, C., Parizek, P., Schwarz, H. P., and Aguzzi, A. (2000). Binding of disease-associated prion protein to plasminogen. Nature 408, 479-483.
Gatti, J. L., Metayer, S., Moudjou, M., Andreoletti, O., Lantier, F., Dacheux, J. L., and Sarradin, P. (2002). Prion protein is secreted in soluble forms in the epididymal fluid and proteolytically processed and transported in seminal plasma. Biol Reprod 67, 393-400.
Heppner, F. L., Musahl, C., Arrighi, I., Klein, M. A., Rulicke, T., Oesch, B., Zinkernagel, R. M., Kalinke, U., and Aguzzi, A. (2001). Prevention of Scrapie Pathogenesis by Transgenic Expression of Anti-Prion Protein Antibodies. Science 294, 178-182.
Horiuchi, M., and Caughey, B. (1999). Specific binding of normal prion protein to the scrapie form via a localized domain initiates its conversion to the protease-resistant state [In Process Citation]. Embo J 18, 3193-3203.
Jeffrey, M., Goodsir, C. M., Bruce, M. E., McBride, P. A., Scott, J. R., and Halliday, W. G. (1992). Infection specific prion protein (PrP) accumulates on neuronal plasmalemma in scrapie infected mice. Neurosci Lett 47, 106-109.
Klein, M. A., Frigg, R., Flechsig, E., Raeber, A. J., Kalinke, U., Bluethmann, H., Bootz, F., Suter, M., Zinkemagel, R. M., and Aguzzi, A. (1997). A crucial role for B cells in neuroinvasive scrapie. Nature 390, 687-690.
Kocisko, D. A., Come, J. H., Priola, S. A., Chesebro, B., Raymond, G. J., Lansbury, P. T., and Caughey, B. (1994). Cell-free formation of protease-resistant prion protein [see comments]. Nature 370, 471-474.
Kocisko, D. A., Priola, S. A., Raymond, G. J., Chesebro, B., Lansbury, P. T., Jr., and Caughey, B. (1995). Species specificity in the cell-free conversion of prion protein to protease-resistant forms: a model for the scrapie species barrier. Proc Natl Acad Sci U S A 92, 3923-3927.
Lansbury, P. T. (1994). Mechanism of scrapie replication Science 265, 1510.
Lund, J., Winter, G., Jones, P. T., Pound, J. D., Tanaka, T., Walker, M. R., Artymiuk, P. J., Arata, Y., Burton, D. R., Jefferis, R., and et al. (1991). Human Fc gamma RI and Fc gamma RII interact with distinct but overlapping sites on human IgG. J Immunol 147, 2657-2662.
Montrasio, F., Frigg, R., Glatzel, M., Klein, M. A., Mackay, F., Aguzzi, A., and Weissmann, C. (2000). Impaired prion replication in spleens of mice lacking functional follicular dendritic cells. Science 288, 1257-1259.
Moullier P, Bohl D, Heard JM, Danos O. (1993). Correction of lysosomal storage in the liver and spleen of MPS VII mice by implantation of genetically modified skin fibroblasts. Nat Genet 4(2): 154-9.
Naslavsky, N., Stein, R., Yanai, A., Friedlander, G., and Taraboulos, A. (1997). Characterization of detergent-insoluble complexes containing the cellular prion protein and its scrapie isoform. J Biol Chem 272, 6324-6331.
Parizek, P., Roeckl, C., Weber, J., Flechsig, E., Aguzzi, A., and Raeber, A. J. (2001). Similar turnover and shedding of the cellular prion protein in primary lymphoid and neuronal cells. J Biol Chem.
Perini, F., Vidal, R., Ghetti, B., Tagliavini, F., Frangione, B., and Prelli, F. (1996). Prp(27-30) Is a Normal Soluble Prion Protein Fragment Released By Human Platelets. Biochemical & Biophysical Research Communications 223, 572-577.
Perrier, V., Kaneko, K., Safar, J., Vergara, J., Tremblay, P., DeArmond, S. J., Cohen, F. E., Prusiner, S. B., and Wallace, A. C. (2002). Dominant-negative inhibition of prion replication in transgenic mice. Proc Natl Acad Sci U S A 99, 13079-13084.
Prusiner, S. B. (1982). Novel proteinaceous infectious particles cause scrapie. Science 216, 136-144.
Prusiner, S. B. (1998). The prion diseases. Brain Pathol 8, 499-513.
Safar, J., Wille, H., Itri, V., Groth, D., Serban, H., Torchia, M., Cohen, F. E., and Prusiner, S. B. (1998). Eight prion strains have PrP(Sc) molecules with different conformations [see comments]. Nat Med 4,1157-1165.
Sailer, A., Büeler, H., Fischer, M., Aguzzi, A., and Weissmann, C. (1994). No propagation of prions in mice devoid of PrP. Cell 77, 967-968.
Schwenk, F., Baron, U., and Rajewsky, K. (1995). A cre-transgenic mouse strain for the ubiquitous deletion of loxP-flanked gene segments including deletion in germ cells. Nucleic Acids Res 23, 5080-5081.
Scott, M., Foster, D., Mirenda, C., Serban, D., Coufal, F., Waelchli, M., Torchia, M., Groth, D., Carlson, G., DeArmond, S. J., *et al.* (1989). Transgenic mice expressing hamster prion protein produce species-specific scrapie infectivity and amyloid plaques. Cell 59, 847-857.
Tao, M. H., Smith, R. I., and Morrison, S. L. (1993). Structural features of human immunoglobulin G that determine isotype-specific differences in complement activation. J Exp Med 178, 661-667.

## Claims

1. A soluble hybrid protein, comprising at least
a) a first polypeptide sequence derived from a prion protein PrP^{c} that is capable of binding a protein responsible for transmissible spongiform encephalitis (PrP^{sc}),
b) and a second polypeptide sequence (tag),
wherein said hybrid protein does not comprise a functional membrane anchor moiety.

2. The hybrid protein of claim 1, which is resistant to conversion to the PrP^{sc} form when binding to PrP^{sc}.

3. The hybrid protein of claim 1 or 2, wherein at least two of said hybrid proteins form multimeric hybrid proteins, preferably dimers or pentamers, more preferably dimers.

4. The hybrid protein according to any one of claims 1 to 3, wherein the second polypeptide sequence is capable of binding to at least one further second polypeptide sequence of at least one further hybrid protein to form multimeric hybrid proteins.

5. The hybrid protein according to any one of claims 1 to 4, wherein the first polypeptide sequence does not comprise the GPI-(Glycosyl-phosphatidyl-Inositol) residue and/or the carboxy-terminal signal sequence of the wild type PrP^{c} prion protein.

6. A multimeric hybrid protein, comprising dimers or oligomers of any one of the hybrid proteins according to any one of claims 1 to 5, preferably dimers and pentamers.

7. The multimeric hybrid protein according to claim 6, wherein the first and the second polypeptide sequence of the hybrid protein are derived from the same or different species.

8. The multimeric hybrid protein according to claim 6 or 7, wherein the monomeric units of the multimeric hybrid proteins are derived from the same or different species.

9. The hybrid protein according to any one of claims 1 to 8, wherein the first poly peptide sequence is a full-length prion protein (PrP^{c}).

10. The hybrid protein according to any one of claims 1 to 9, wherein said second polypeptide sequence is derived from an immunoglobulin.

11. The hybrid protein of claim 10, wherein in the second polypeptide the binding sites for Fc_{γ} receptors and/or complement are inactivated.

12. The hybrid protein according to any one of claims 1 to 11, wherein said second polypeptide sequence is derived from an immunoglobulin heavy chain constant region.

13. The hybrid protein of claim 12, wherein said second polypeptide sequence is derived from a mammalian immunoglobulin gamma heavy chain, preferably from the human immunoglobulin gamma heavy chain.

14. The hybrid protein of claim 12, wherein said second polypeptide sequence is derived from a mammalian immunoglobulin mµ heavy chain, preferably from the human immunoglobulin mµ heavy chain.

15. The hybrid protein of any one of claims 1 to 14, wherein said second polypeptide sequence is fused directly to the carboxy-terminal side of the first polypeptide sequence.

16. The hybrid protein according to any one of claims 1 to 15, wherein the first polypeptide sequence is a functional derivative of the human prion protein (PrP^{c}) or a functional fragment thereof, comprising the PrP^{sc} binding domain of human PrP^{c} that is preferably at least 50%, more preferably at least 80%, and most preferably at least 90% identical to the PrP^{sc} binding domain of the human naturally occurring, fully functional, wild-type prion protein PrP^{c}, and retains at least some, preferably at least 20%, more preferably at least 50%, and most preferably at least 90% of the specific binding capability to a PrP^{sc}-protein of the PrP^{sc} binding domain of the wild-type prion protein

17. The hybrid protein according to claim 16, wherein the functional derivative of the wild-type prion protein is a functional fragment, variant, analog, chemical derivative, or peptidomimetic of the wild-type prion protein or a functional fragment thereof.

18. A hybrid protein according to any one of claims 1 to 17, wherein said protein is bound to a solid support.

19. Use of a hybrid protein according to any one of claims 1 to 18 for the diagnosis of transmissible spongiform encephalopathies (TSEs).

20. Diagnostic kits for use in the diagnosis of transmissible spongiform encephalopathies (TSEs) and containing a protein according to any one of claim 1 to 18 in combination with further reagents, such as buffers, and working instructions.

21. Process for the detection of transmissible spongiform encephalopathies (TSEs) comprising
(a) contacting a sample suspected of comprising a PrP^{sc} protein with a hybrid protein according to any one of claims 1 to 18 under conditions that allow for the binding of the PrP^{sc} protein to the hybrid protein, and
(b) detecting the PrP^{sc} protein-hybrid protein complex.

22. Process for the identification and/or isolation of molecules capable of binding to PrP^{sc} and/or PrP^{c} comprising
(d) contacting a sample suspected of comprising a PrP^{sc} and/or PrP^{c} binding molecule with a hybrid protein according to any one of claims 1 to 18 under conditions that allow for the binding of the molecule to the hybrid protein either directly or indirectly, and
(e) detecting whether the hybnd protein has bound a molecule, and optionally
(f) isolating the molecule directly or indirectly bound to the hybrid protein.

23. Use of a hybrid protein according to any one of claims 1 to 18 for the identification of molecules capable of binding to PrP^{sc} and/or PrP^{c}.

24. Nucleic acid encoding a hybrid protein according to any one of claims 1 to 18.

25. Vector comprising a nucleic acid according to claim 24.

26. Host cell, comprising a nucleic acid according to claim 24 and/or a vector according to claim 25.

27. Use of a hybrid protein according to any one of claims 2 to 18, a nucleic acid encoding a hybrid protein according to any one of claims 2 to 18, a vector, and/or a host cell comprising a nucleic acid encoding a hybrid protein according to any one of claims 2 to 18 for the preparation of a medicament for the prevention and/or treatment of transmissible spongiform encephalopathies (TSEs).

28. A pharmaceutical composition comprising a hybrid protein according to any one of claims 2 to 18, a nucleic acid encoding a hybrid protein according to any one of claims 2 to 18, a vector, and/or a host cell comprising a nucleic acid encoding a hybrid protein according to any one of claims 2 to 18, and a pharmaceutically acceptable carrier.

29. The pharmaceutical composition according to claim 28 for the use in gene therapy.

30. A process for producing a hybrid protein according to any one of claims 1 to 18 comprising the step of culturing a host cell according to claim 26 under conditions that allow for the expression of said hybrid protein.

31. A transgenic animal, preferably a transgenic mouse, stably expressing a hybrid protein according to any one of claims 1 to 18.

32. Bone marrow of a transgenic animal according to claim 31.

33. The use of bone marrow according to claim 32 for the preparation of a medicament for the treatment of transmissible spongiform encephalopathies (TSEs).

34. A composition comprising a PrP^{sc} and a hybrid protein according to any one of claims 1 to 18.

35. The composition according to claim 34, wherein PrP^{sc} is bound to the hybrid protein.

36. The composition according to claim 35, wherein PrP^{sc} is noncovalently bound to the hybrid protein

37. A carrier comprising a hybrid protein according to any one of claims 1 to 18.

38. The carrier according to claim 37 which is selected from magnetic beads, filter stripes, microtiter plates, non-magnetic beads, plasmon surface resonance plates, microarray plates, liquid carriers undergoing phase transition to solid, and combination thereof.

39. An *in vitro* process of removing PrP^{sc} from biological material, comprising the step of contacting the material with a hybrid protein according to any one of claims 1 to 18 and removing PrP^{sc} bound to the hybrid protein.

40. Use of a hybrid protein according to any one of claims 1 to 18, and/or a composition according to any one of claims 34 to 36, and/or a carrier according to claim 37 or 38 for removing and/or inactivating PrP^{sc} from a biological material.


Sequence Listing

<110> UNIVERSITY OF ZUERICH

<120> Soluble hybrid prion proteins and their use in the diagnosis, prevention
and treatment of transmissible spongiform encephalopathies

<130> EP27008-03138/GRI

<140> EP03005829.1
<141> 2003-03-14

<160> 2

<170> PatentIn Ver. 3.1

<210> 1
<211> 24bp
<212> DNA
<213> Artificial Sequence

<220>
<223> primer 602

<400> 1
CAGAACTGAA CCATTTCAAC CGAG 24

<210> 2
<211> 25bp
<212> DNA
<213> Artificial Sequence

<220>
<223> primer 603

<400> 2
TCAGTCCACA TAGTCACAAA GAGGG 25